# EUROPEAN PATENT APPLICATION

(11) **EP 3 995 480 A1**
(43) Date of publication of application: **11.05.2022**
(21) Application number: 20834153.7
(22) Date of filing: 30.06.2020
(51) Int. Cl.: C07C 33/20, C07C 205/38, C07F 9/22, C07F 9/564, C07B 55/00, A61K 31/04, A61K 31/06, A61K 31/664, A61P 35/00

(54) **AKR1C3 INHIBITOR AND MEDICAL USE**

(30) Priority: 01.07.2019 CN 201910585317; 20.12.2019 CN 201911323885
(71) Applicant: Ascentawits Pharmaceuticals, Ltd., Shenzhen, Guangdong 518118 (CN)
(72) Inventor: DUAN, Jianxin, Shenzhen, Guangdong 518118 (CN); LI, Anrong, Shenzhen, Guangdong 518118 (CN); MENG, Fanying, San Francisco, California 94121 (US); CAI, Xiaohong, Shenzhen, Guangdong 518118 (CN)
(74) Representative: Haseltine Lake Kempner LLP
(86) International application number: PCT/CN2020/099353
(87) International publication number: WO 2021/000862

(57) **Abstract**

The present invention provides an AKR1C3 inhibitor of compounds of the following formulas and pharmaceutically acceptable salts or solvates or isotopically substituted compounds thereof and medical use.

## Description

### TECHNICAL FIELD

The present application relates to an AKR1C3 inhibitor and the medical use thereof.

### BACKGROUND

DNA alkylating agents, prodrugs of the compounds for treating cancer (Application No. PCT/US2016/021581, Publication No. WO2016/145092; Application No. PCT/US2016/062114, Publication No. WO2017/087428) developed by our company that target the highly expressed aldoketo reductase 1C3 (AKR1C3) are specifically metabolically activated under the action of AKR1C3 in vivo, exemplified by AST-3424, which is the S configuration of TH-2870:

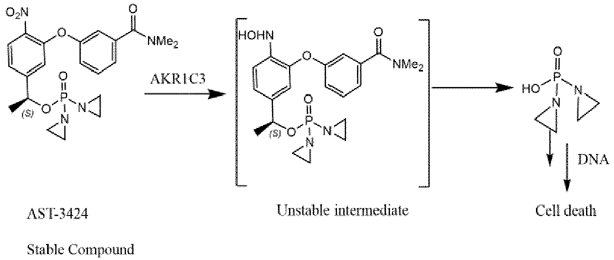

The DNA alkylating agent that targets the highly expressed aldoketo reductase AKR1C3 in the form of prodrug will bind to AKR1C3 in vivo and then undergo metabolic reactions, ultimately resulting in DNA alkylating agents that are cytotoxic.

### SUMMARY

In the attempts to perform individual group substitution for such compounds during the research and development, it was discovered that when what is attached to the carbon atom of the benzyl on the para position of the nitro group on the phenyl ring is not similar to the cytotoxic alkylating agents as described in Application No. PCT/US2016/021581, Publication No. WO2016/145092; Application No. PCT/US2016/062114, Publication No. WO2017/087428; Application No. PCT/US2016/025665, Publication No. WO2016/161342, the compounds exhibit the ability to inhibit the enzymatic activity of AKR1C3. Therefore, the research and development team designed and synthesized a series of AKR1C3 inhibitors of new chemical structures.

To this end, the present application provides the following technical solutions.

A compound of the following formula and a pharmaceutically acceptable salt, or a solvate, or an isotopically substituted compound thereof: 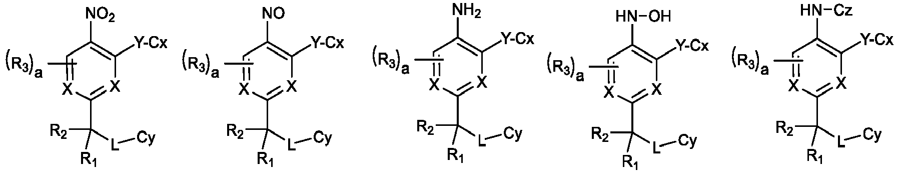
wherein, R₁ and R₂ are each independently hydrogen, deuterium, aryl or Z-substituted aryl, heteroaryl or Z-substituted heteroaryl, C₁-C₆ alkyl or Z-substituted alkyl, C₂-C₆ alkenyl or Z-substituted alkenyl, C₂-C₆ alkynyl or Z-substituted alkynyl, or C₃-C₈ cycloalkyl or Z-substituted cycloalkyl;
R₃ is hydrogen, halogen, cyano or isocyanato, hydroxyl, mercapto, amino, oximido, hydrazono, OTs, OMs, C₁-C₆ alkyl or Z-substituted alkyl, C₂-C₆ alkenyl or Z-substituted alkenyl, C₂-C₆ alkynyl or Z-substituted alkynyl, C₃-C₈ cycloalkyl or Z-substituted cycloalkyl, C₆-C₁₀ aryl or Z-substituted aryl, 4-15-membered heterocyclic radical or Z-substituted heterocyclic radical, 5-15-membered heteroaryl or Z-substituted heteroaryl, C₁-C₆ alkoxy or Z-substituted C₁-C₆ alkoxy; or R₃ is -CONR⁶R⁷, -SO₂NR⁶R⁷, -SO₂R⁶, -OCO-R⁶, -OCOO-R⁶, - COOR⁶, -NR⁶COR⁷, -NR⁶SO₂R⁷, or -NR⁶CONR⁶R⁷, and R⁶ and R⁷ together with N to form or not to form a 4-8-membered Z-substituted heterocyclic ring;
R⁶ and R⁷ are each independently hydrogen, cyano or isocyanato, C₁-C₆ alkyl or Z-substituted alkyl, C₂-C₆ alkenyl or Z-substituted alkenyl, C₂-C₆ alkynyl or Z-substituted alkynyl, C₃-C₈ cycloalkyl or Z-substituted cycloalkyl, C₆-C₁₀ aryl or Z-substituted aryl, 4-15-membered heterocyclic radical or Z-substituted heterocyclic radical, 5-15-membered heteroaryl or Z-substituted heteroaryl, or C₁-C₆ alkoxy or Z-substituted C₁-C₆ alkoxy, or R⁶ and R⁷ together with the atom to which they are bonded to form a 3-7-membered heterocyclic radical or Z-substituted 3-7-membered heterocyclic radical;
a is 0, 1, 2, or 3;
X is C or N;
in the above chemical formulae, R₃ may be located on different atoms of the benzene ring or the pyridine ring. For example, there may be no R₃ group, in which case a is 0, i.e., there is hydrogen on the benzene ring or pyridine ring; there may be one R₃ group, in which case a is 1, i.e., the remaining 2 (in the case of pyridine ring) or 3 (in the case of benzene ring) hydrogen atoms on the benzene ring or pyridine ring are substituted by one R₃ group; there may be two R₃ groups, in which case a is 2, i.e., the remaining 2 (in the case of pyridine ring) or 3 (in the case of benzene ring) hydrogen atoms on the benzene ring or pyridine ring are substituted by two R₃ groups; or there may be three R₃ groups, in which case a is 3, i.e., the three hydrogen atoms on the phenyl ring are substituted by three R₃ groups.
Y is O or S;
Cx is selected from the group consisting of C₆-C₁₀ aryl or Z-substituted aryl, 4-15-membered heterocyclic radical or Z-substituted 4-15-membered heterocyclic radical, 5-15-membered heteroaryl or Z-substituted heteroaryl, 7-15-membered fused ring or Z-substituted fused ring, and -CONR⁶R⁷, -SO₂NR⁶R⁷, -SO₂R⁶, - OCOO-R⁶, -COOR⁶, -NR⁶COR⁷, -OCOR⁶, -NR⁶SO₂R⁷, -NR⁶SO₂NR⁶R⁷, -COR⁶, -NR⁶CONR⁶R⁷ substituted C₆-C₁₀ aryl, 4-15-membered heterocyclic radical, 5-15-membered heteroaryl, and 7-15-membered fused ring; and R⁶ and R⁷ together with N to form or not to form a 4-8-membered Z-substituted heterocycle;
L is selected from the group consisting of -O-, -S-, -OCOO-, -NR⁶CO-, -OCO-, -NR⁶SO₂-, -OCONR⁶-, quaternary ammonium, and sulfonate group -OSO₂-;
Cy is selected from the group consisting of hydrogen, deuterium, C₆-C₁₀ aryl or Z-substituted aryl, 4-15-membered heterocyclic radical or Z-substituted heterocyclic radical, 5-15-membered heteroaryl or Z-substituted heteroaryl, 7-15-membered fused or Z-substituted fused ring, C₁-C₆ alkyl or Z-substituted alkyl, C₂-C₆ alkenyl or Z-substituted alkenyl, C₂-C₆ alkynyl or Z-substituted alkynyl, or C₃-C₈ cycloalkyl or Z-substituted C₃-C₈ cycloalkyl;
   or
Cy is selected from the group consisting of 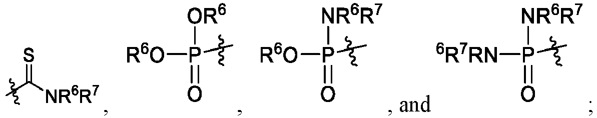
or Cy is selected from the group consisting of a 5-10-membered ring group formed by the two OR⁶ and the P atom in 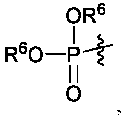 a 5-10-membered ring group formed by the OR⁶ and the NR⁶R⁷ together with the P atom in 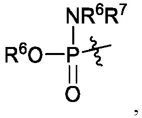 and a 5-10-membered ring group formed by the two NR⁶R⁷ and the P atom in 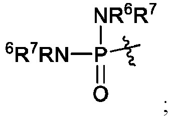
and -L-Cy excludes the residues of these phosphoramidate alkylating agents after losing a H atom: - P(Z¹)(NR⁹CH₂CH₂X¹)₂, -P(Z¹)(NR⁹₂)(N(CH₂CH₂X¹)2), -P(Z¹)(N(CH₂CH₂X¹))₂ or-P(Z¹)(N(CH₂CH₂X¹)₂)₂, each R⁹ is independently hydrogen or C₁-C₆ alkyl, or two R⁹ together with the nitrogen atom to which they are bonded to form a 5-7-membered heterocyclyl, Z¹ is O or S, X¹ is Cl, Br or OMs, and -L-Cy excludes -OH or -SH;
the substituent Z is a halogen atom, cyano or isocyanato, hydroxyl, mercapto, amino, oximido, hydrazono, OTs, OMs, C₁-C₃ alkyl or substituted alkyl, C₁-_{C3} alkoxy or substituted alkoxy, C₂-C₃ alkenyl or substituted alkenyl, C₂-C₃ alkynyl or substituted alkynyl, C₃-C₈ cycloalkyl or substituted cycloalkyl, an aromatic ring, heterocyclic ring, a heteroaromatic ring and fused ring or a substituted aromatic ring, heterocyclic ring, heteroaromatic ring and fused ring, and the pattern of substitution being mono-substitution or geminal di-substitution;
Cz group is a C-, P-, S-containing group which can be hydrolyzed by hydrolytic enzymes such that corresponding C-N, P-N or S-N bond is cleaved.

A compound of the following formula I or a pharmaceutically acceptable salt, or a solvate, or an isotopically substituted compound thereof, 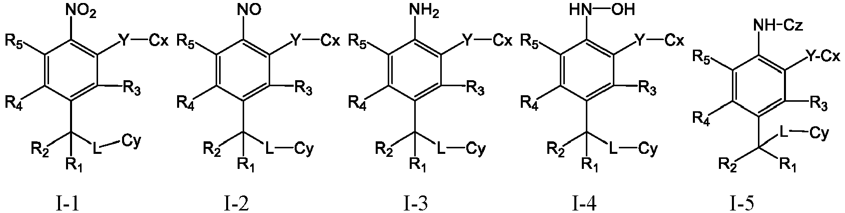
or a prodrug that can be converted *in vivo* to the above compound of formula 1-3 thereof,
wherein,
R₁ and R₂ are each independently hydrogen, deuterium, aryl or Z-substituted aryl, heteroaryl or Z-substituted heteroaryl, C₁-C₆ alkyl or Z-substituted alkyl, C₂-C₆ alkenyl or Z-substituted alkenyl, C₂-C₆ alkynyl or Z-substituted alkynyl, or C₃-C₈ cycloalkyl or Z-substituted cycloalkyl;
R₃ is hydrogen, halogen, cyano or isocyanato, hydroxyl, mercapto, amino, oximido, hydrazono, OTs, OMs, C₁-C₆ alkyl or Z-substituted alkyl, C₂-C₆ alkenyl or Z-substituted alkenyl, C₂-C₆ alkynyl or Z-substituted alkynyl, C₃-C₈ cycloalkyl or Z-substituted cycloalkyl, C₆-C₁₀ aryl or Z-substituted aryl, 4-15-membered heterocyclic radical or Z-substituted heterocyclic radical, 5-15-membered heteroaryl or Z-substituted heteroaryl, or C₁-C₆ alkoxy or Z-substituted C₁-C₆ alkoxy; or R₃ is -CONR⁶R⁷, -SO₂NR⁶R⁷, -SO₂R⁶, -OCO-R⁶, -OCOO-R⁶, - COOR⁶, -NR⁶COR⁷, -NR⁶SO₂R⁷, or -NR⁶CONR⁶R⁷, and R⁶ and R⁷ together with N to form or not to form a 4-8-membered Z-substituted heterocyclic ring;
R₄ and R₅ are each independently hydrogen, halogen, cyano or isocyano, hydroxy, mercapto, amino, oximido, hydrazono, OTs, OMs, C₁-C₆ alkyl or Z-substituted alkyl, C₂-C₆ alkenyl or Z-substituted alkenyl, C₂-C₆ alkynyl or Z-substituted alkynyl, C₃-C₈ cycloalkyl or Z-substituted cycloalkyl, C₆-C₁₀ aryl or Z-substituted aryl, 4-15-membered heterocyclic radical or Z-substituted heterocyclic radical, 5-15-membered heteroaryl or Z-substituted heteroaryl, or C₁-C₆ alkoxy or Z-substituted C₁-C₆ alkoxy; or R₄ and R₅ are each -CONR⁶R⁷, - SO₂NR⁶R⁷, -SO₂R⁶, -OCOO-R⁶, -COOR⁶, -NR⁶COR⁷, -OCOR⁶, -NR⁶SO₂R⁷, or -NR⁶CONR⁶R⁷, or R₄ and R₅ together with the atom on a benzene ring to which they are bonded to form a 7-15-membered fused ring or a Z-substituted fused ring, and R⁶ and R⁷ together with N to form or not to form a 4-8-membered Z-substituted heterocyclic ring;
R⁶ and R⁷ are each independently hydrogen, cyano or isocyanato, C₁-C₆ alkyl or Z-substituted alkyl, C₂-C₆ alkenyl or Z-substituted alkenyl, C₂-C₆ alkynyl or Z-substituted alkynyl, C₃-C₈ cycloalkyl or Z-substituted cycloalkyl, C₆-C₁₀ aryl or Z-substituted aryl, 4-15-membered heterocyclic radical or Z-substituted heterocyclic radical, 5-15-membered heteroaryl or Z-substituted heteroaryl, or C₁-C₆ alkoxy or Z-substituted C₁-C₆ alkoxy; or R⁶ and R⁷ together with the atom to which they are bonded to form a 3-7-membered heterocyclic radicalor a Z-substituted 3-7-membered heterocyclic radical, and R⁶ and R⁷ together with N to form or not to form a 4-8-membered Z-substituted heterocyclic ring;
Y is O or S;
Cx is selected from the group consisting of C₆-C₁₀ aryl or Z-substituted aryl, 4-15-membered heterocyclic radical or Z-substituted 4-15-membered heterocyclic radical, 5-15-membered heteroaryl or Z-substituted heteroaryl, 7-15-membered fused ring or Z-substituted fused ring, and -CONR⁶R⁷, -SO₂NR⁶R⁷, -SO₂R⁶, - OCOO-R⁶, -COOR⁶, -NR⁶COR⁷, -OCOR⁶, -NR⁶SO₂R⁷, -NR⁶SO₂NR⁶R⁷, -COR⁶, -NR⁶CONR⁶R⁷ substituted C₆-C₁₀ aryl, 4-15-membered heterocyclic radical, 5-15-membered heterocyclic radical, 7-15-membered fused ring, and R⁶ and R⁷ together with N to form or not to form a 4-8-membered Z-substituted heterocycle;
L is selected from -O-, -S-, -OCOO-, -NR⁶CO-, -OCO-, -NR⁶SO₂-, -OCONR⁶⁻, quaternary ammonium, and sulfonate group -OSO₂-;
Cy is selected from the group consisting of hydrogen, deuterium, C₆-C₁₀ aryl or Z-substituted aryl, 4-15-membered heterocyclic radical or Z-substituted heterocyclic radical, 5-15-membered heteroaryl or Z-substituted heteroaryl, 7-15-membered fused ring or Z-substituted fused ring, C₁-C₆ alkyl or Z-substituted alkyl, C₂-C₆ alkenyl or Z-substituted alkenyl, C₂-C₆ alkynyl or Z-substituted alkynyl, or C₃-C₈ cycloalkyl or Z-substituted C₃-C₈ cycloalkyl;
   or
Cy is selected from the group consisting of 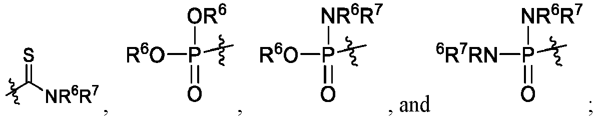
or Cy is selected from the group consisting of a 5-10-membered ring group formed by the two OR⁶ and the P atom in 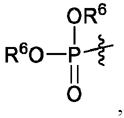 a 5-10-membered ring group formed by the OR⁶ and the NR⁶R⁷ and together with the P atom in 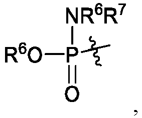 and a 5-10-membered ring group formed by the two NR⁶R⁷and the P atom in 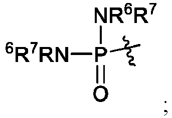
and the above H-L-Cy does not form a phosphate alkylating agent;
the substituent Z is a halogen atom, cyano or isocyanato, hydroxyl, mercapto, amino, oximido, hydrazono, OTs, OMs, C₁-C₃ alkyl or substituted alkyl, C₁-C₃ alkoxy or substituted alkoxy, C₂-C₃ alkenyl or substituted alkenyl, C₂-C₃ alkynyl or substituted alkynyl, C₃-C₈ cycloalkyl or substituted cycloalkyl, an aromatic ring, heterocyclic ring, a heteroaromatic ring and fused ring or a substituted aromatic ring, heterocyclic ring, heteroaromatic ring and fused ring, and the pattern of substitution being mono-substitution or geminal di-substitution;
Cz group is a C-, P-, S-containing group which can be hydrolyzed by hydrolytic enzymes such that corresponding C-N, P-N or S-N bond is cleaved, i.e., Cz is an amino acid residue (-NH-Cz forms an amino acid peptide bond), an organic carboxylic acid residue (-NH-Cz forms an amide structure), etc.

Hydrolases are classified as EC3 in EC number and subdivided into several subclasses by the bond it breaks down:
EC3.1: ester bond (esterase)
EC3.2: sugar (glycosylase)
EC3.3: ether linkage
EC3.4: peptide bond (peptidase)
EC3.5: C-N bond, but excluding peptide bond
EC3.6: anhydride
EC3.7: C-C bond
EC3.8: halogen bond
EC3.9: P-N bond
EC3.10: S-N bond
EC3.11: S-P bond
EC3.12: S-S bond
EC3.13: C-S bond

In the human physiological environment, there are many enzymes capable of cleaving the -NH-Cz in the prodrugs described above. In particular, for example, hydrolases are capable of cleaving many chemical bonds by hydrolyzation, particularly the groups containing C, P and S atoms in the compounds disclosed herein. In particular, these chemical bonds and hydrolases include EC3.4: peptide bonds (peptidases), EC3.9: P-N bonds and EC3.10: S-N bonds, and the corresponding compounds include amides, phosphoramides and thioamides.

Heterocycle and heteroaryl include three-membered rings, four-membered rings, five-membered rings, six-membered rings, and seven-membered rings. The examples are given below.
The three-membered rings include ethylene oxide, azirane and ethylene sulfide;
the four-membered rings include azetidine, oxaetidine, thiaetidine and etidine;
the five-membered rings include pyrrolidine, pyrroline, 1-pyrroline, 3-pyrroline, 2-pyrroline, pyrrole, pyrazolidine, 2-pyrazoline, imidazole, pyrazole, furan, tetrahydrofuran, dihydrofuran, tetrahydrothiophene, thiophene, sulfolane, phosphole, oxazole, 1, 2, 3-triazole, 1, 2, 4-triazole, and 1, 3, 4-thiadiazole;
the six-membered rings include piperidine, tetrahydropyran, tetrahydrothiopyran, pyridine, pyran, thiopyran, dihydropyridine, morpholine, piperazine, pyridazine, pyrazine, 1, 3, 5-triazine, and 1, 3, 5-trithiane;
the seven-membered rings include azepane (azacycloheptane), oxaheptane, thiaheptane, azepine, oxepine, and thiepine.

The fused ring is defined as the fusion of the above heterocycle and heteroaryl or the fusion of the above heterocycle and heteroaryl with a cycloalkane structure. The fusion can be in the form of binding via a single bond or in the form of sharing one, two, or even three atoms. Some common fused ring structures are provided below: naphthalene, quinoline, indole, isoindole, isoquinoline, cinnoline, quinoxaline, biphenyl, coumarin, fluorene, diphenylcarran, carbazole, anthracene, acridine, thiophenazine, adamantane, azulene, phenanthrene, anthraquinone, flavone, and isoflavone.

Apparently, the above compounds also include isotopically substituted compounds. The typical pattern of substitution is that a hydrogen atom H is substituted by a heavy hydrogen atom deuterium D.

In particular, the position substituted by deuterium is located on Ph-C^{∗}- in formula I as shown in the following formulae:

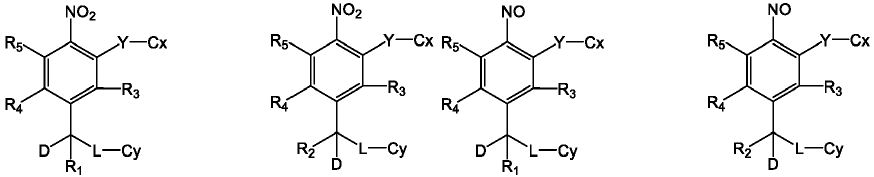

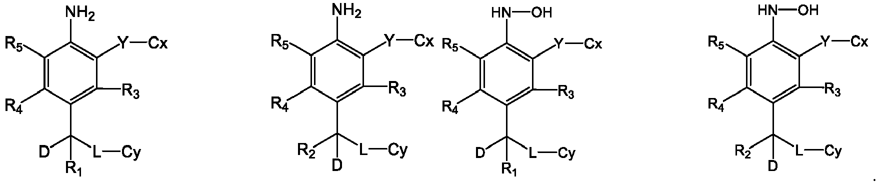

In a further embodiment, in the compounds provided above, the salt is a basic salt or an acid salt.

With respect to the compounds described herein, the compounds also include the form of their salts of the structures of Formulae 1-1 to 1-5. In other words, the present application provides pharmaceutically acceptable salts of the compounds as shown herein. The salts may be basic salts, including the salts of the compounds formed with inorganic bases (such as alkali metal hydroxides, or alkaline earth metal hydroxides, and the like), or with organic bases (such as mono-, di-, or triethanolamine, and the like). Alternatively, the salts may be acid salt, including the salts of the compounds formed with inorganic acids (such as hydrochloric acid, hydrobromic acid, hydroiodic acid, nitric acid, perchloric acid, sulfuric acid, or phosphoric acid, or the like), or with organic acids (such as methanesulfonic acid, trifluoromethanesulfonic acid, ethanesulfonic acid, benzenesulfonic acid, p-toluenesulfonic acid, fumaric acid, oxalic acid, maleic acid, or citric acid, and the like). It is a well-known technology in the art to select and prepare an acceptable salt and a solvate, and the like of a compound.

In a further embodiment, regarding the compounds provided above, the solvate is a hydrate or alcoholate.

In the technical field of drug design and organic chemistry, with regard to the compounds, in particular compounds with more reactive groups, such as phosphoramides, amides, amines, salts or esters, due to a better affinity to solvents such as water and alcoholic solvents such as ethanol, a conjugate of a solvent and a compound (typically an inorganic salt such as copper sulfate hydrate) is often generated. In particular, when the compound is a solid obtained from a solvent by crystallization, precipitation, concentration or the like, the compound will inevitably result in a solvate which is conjugated with the solvent as a result of being combined and encapsulated with the solvent. Since the compounds provided herein have reactive groups such as phosphoramide, amide, hydroxyl, and the like, naturally the corresponding solvates can be generated for the reasons and practices described above.

The compounds described herein may also be used in the form of solvates. In other words, the present application provides pharmaceutically acceptable solvates of the compounds of formula I. The solvate is a hydrate, an alcoholate and the like, wherein the alcoholate includes ethanolates.

It has been confirmed through experiments that the above compound I has the activity of inhibiting AKR1C3 enzyme

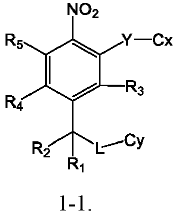

Literature investigations have shown that this class of compounds undergoes the following metabolic reactions under the action of the AKR1C3 enzyme:

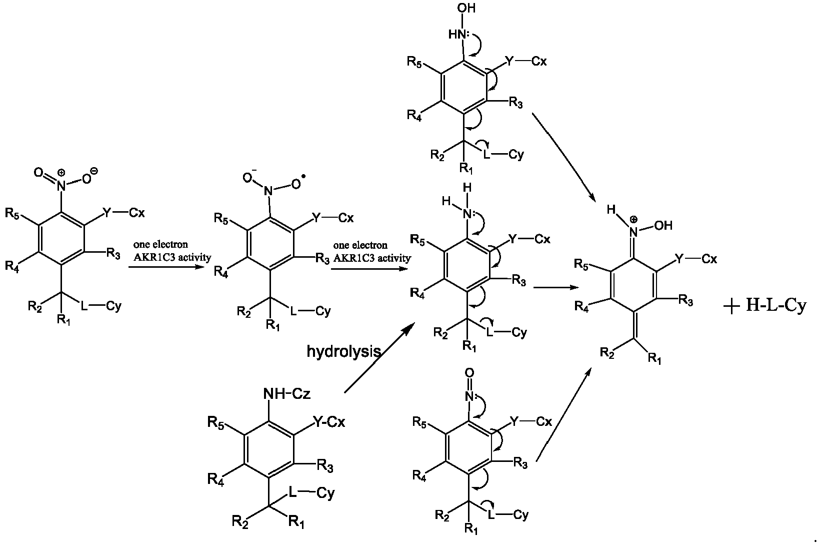

The intermediates of the following three structures (Roger M. Phillips, Targeting the hypoxic fraction of tumours using hypoxia-activated prodrugs[J]. Cancer Chemother Pharmacol (2016) 77:441-457.DOI 10.1007/s00280-015-2920-7; Baran N, Konopleva M.Molecular Pathways:Hypoxia-activated prodrugs in cancer therapy[J].Clinical Cancer Research, 2017:clincanres.0895.2016.DOI: 10.1158/1078-0432.CCR-16-0895; Cindy Cazares-Körner, Isabel M.Pires, I.Diane Swallow, et al. CH-01 is a Hypoxia-Activated Prodrug That Sensitizes Cells to Hypoxia/Reoxygenation Through Inhibition of Chk1 and Aurora A[J].ACS Chem.Biol.2013, 8(7):1451.DOI:10.1021/cb4001537; Potent and Highly Selective Hypoxia-Activated Achiral Phosphoramidate Mustards as Anticancer Drugs[J]. Journal of Medicinal Chemistry, 2008, 51(8):2412-2420.DOI:10.1021/jm701028q; Jameson M B, Rischin D, Pegram M, et al. A phase I trial of PR-104, a nitrogen mustard prodrug activated by both hypoxia and aldo-keto reductase 1C3, in patients with solid tumors[J]. Cancer Chemotherapy & Pharmacology, 2010, 65(4):791-801.DOI:10.1007/s00280-009-1188-1; Hunter F W, Wouters B G, Wilson W R . Hypoxia-activated prodrugs: paths forward in the era of personalised medicine[J]. British Journal of Cancer, 2016.DOI:10.1038/bjc.2016.79; Pre-clinical activity of PR-104 as monotherapy and in combination with sorafenib in hepatocellular carcinoma[J]. Cancer Biology & Therapy, 2015, 16(4):610-622.DOI: 10.1080/15384047.2015.1017171) can be obtained from the nitro compound of formula I above during two reductions under the action of the AKR1C3 enzyme

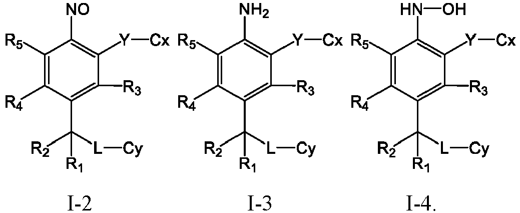

Therefore, the three intermediates (nitroso, hydroxylamine, and amine group) can correspondingly exert an AKR1C3 enzyme inhibition activity similar to that of the nitro compound of formula I.

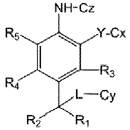

Since the prodrug I-5 can be hydrolyzed by various enzymes (amidohydrolases, phosphoamidohydrolases) in vivo to obtain a corresponding amine form, i.e., the compound of I-3, the prodrugs 1-5 and 1-3 similarly function as AKR1C3 inhibitors in vivo.

In a further embodiment, R₁ and R₂ are each independently hydrogen, deuterium, C₁-C₆ alkyl or Z-substituted alkyl, C₂-C₆ alkenyl or Z-substituted alkenyl, C₂-C₆ alkynyl or Z-substituted alkynyl, or C₃-C₈ cycloalkyl or Z-substituted cycloalkyl.

In a further embodiment, among them, R₁ and R₂ are each independently hydrogen, deuterium, or methyl.

In a further embodiment, among them, R₃, R₄ and R₅ are each independently hydrogen.

In a further embodiment, among them, Cx is -CONR⁶R⁷ substituted phenyl, and R⁶ and R⁷ together with N to form or not to form a 4-8-membered Z-substituted heterocyclic ring.

In a further embodiment, among them, L is selected from -O-, or -S-.

In a further embodiment, among them, Cy is selected from the group consisting of C₆-C₁₀ aryl or halo-substituted aryl, 4-15-membered heterocyclic radical or halo-substituted heterocyclic radical, 5-15-membered heteroaryl or halo-substituted heteroaryl, and 7-15-membered fused ring or halo-substituted fused ring.

In a further embodiment, among them, Cy is selected from the group consisting of fluorophenyl, difluorophenyl, and trifluorophenyl.

In a further embodiment, among them, Cy is selected from the group consisting of 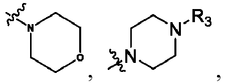 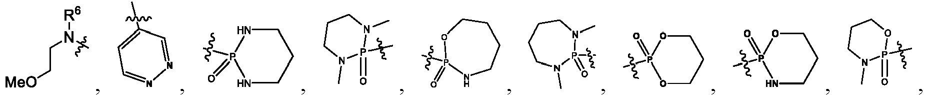 and 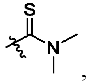 or Z-substituted

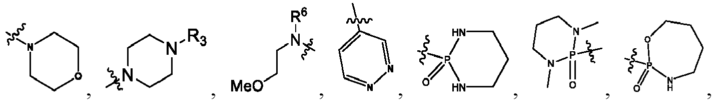

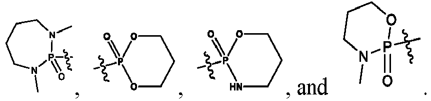

In a further embodiment, among them, the prodrug 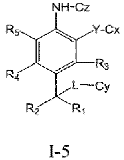 is selected from 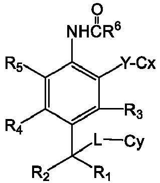 or 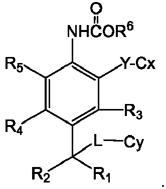 That is, Cz in the prodrug 1-5 in this case is -COR⁶ or -COOR⁶.

In a further embodiment, among them, -NH-Cz is a phosphamido group.

In a further embodiment, the compound is selected from the compounds of the following structures:

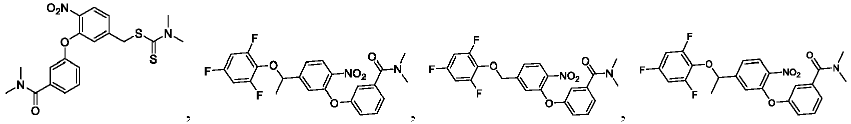

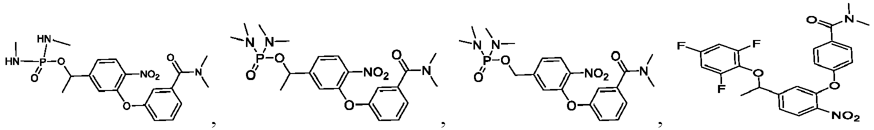

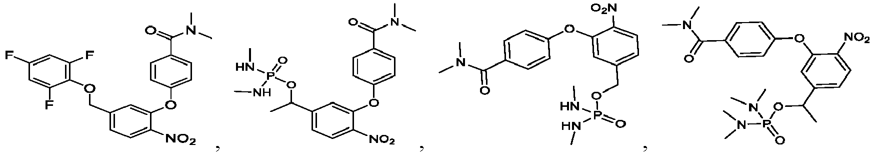

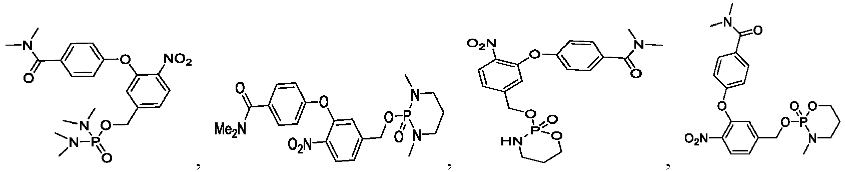

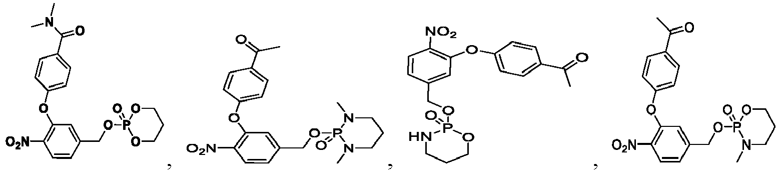

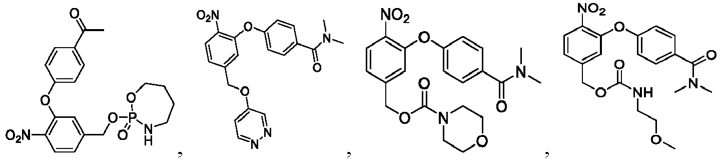

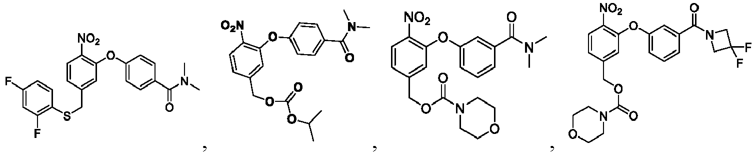

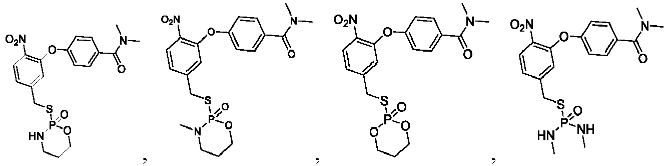

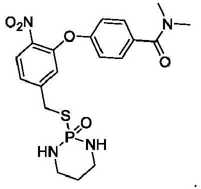

Wherein, the salt is a basic salt or an acid salt, and the solvate is a hydrate or an alcoholate.

Through literature research, it can be known from the disclosure of Taiwan Patent Document TW201742868A (published on December 16, 2017, the contents of which are also disclosed in WO2017202817A1, AU2017269871A1, US20180319807A2, US20170342082A1, etc.) that:
The aldo-keto reductase family 1 member C3 (AKR1C3, also known as type 5 17-beta- hydroxysteroid dehydrogenase (17-β-HSD5)) is a member of the aldo-keto reductase (AKR) superfamily of enzymes, and it reduces the aldehyde/keto group in steroid hormones to the corresponding alcohol and therefore play an important role in the metabolism/activation/deactivation of androgen, progesterone, estrogene and prostaglandin.

AKR1C3 possesses the activities of 3α-HSD (hydroxysteroid dehydrogenase), 17β-HSD, 20α-HSD and prostaglandin (PG) F synthase. It catalyzes the conversion of estrone (which possesses weak estrogenic activity) to estradiol (which possesses potent estrogenic activity), the conversion of progesterone (which possesses potent anti-estrogenic activity) to 20-α-hydroxyprogesterone (weak anti-estrogenic activity) and the conversion of androstenedione to testosterone (Labrie F, Luu-The V, Labrie C, et al. DHEA and Its Transformation into Androgens and Estrogens in Peripheral Target Tissues: Intracrinology[J]. Frontiers in Neuroendocrinology, 2001, 22(3):185-212.DOI: 10.1006/frne.2001.0216). Furthermore, AKR1C3 catalyzes the conversion of PGH2 to PGF2α and the conversion of PGD2 to 11β-PGF2, both are known to stimulate inflammation and proliferation.

Furthermore, it has also been disclosed that AKR1C3 may metabolize a wide range of carbonyl compounds and xenobiotics, including clinically administered anthracyclines (Bains O S, Grigliatti T A, Reid R E, et al. Naturally occurring variants of human aldo-keto reductases with reduced in vitro metabolism of daunorubicin and doxorubicin.[J]. Journal of Pharmacology & Experimental Therapeutics, 2010, 335(3):533-545.DOI: 10.1124/jpet.110.173179; Novotna R, Wsol V, Xiong G, et al. Inactivation of the anticancer drugs doxorubicin and oracin by aldo-keto reductase (AKR) 1C3[J]. Toxicology Letters, 2008, 181(1):1-6.DOI:10.1016/j.toxlet.2008.06.858; Hofman J, Malcekova B, Skarka A, et al. Anthracycline resistance mediated by reductive metabolism in cancer cells: The role of aldo-keto reductase 1C3[J]. Toxicology and Applied Pharmacology, 2014, 278(3):238-248.DOI: 10.1016/j.taap.2014.04.027).

AKR1C3 is closely related with endometriosis. AKR1C3 plays a role in several pathologic conditions/diseases, which include endometriosis.

Endometriosis is a chronic, mainly estrogen-dependent inflammatory disease, which characterized by the presence of endometrial tissue outside the uterine cavity. The major symptoms of endometriosis are chronic pelvic pain and subfertility. Estrogen (E2) deprivation is the clinically proven primary mechanism of action for pharmacological treatment of endometriosis. Besides systemic estrogen levels, there is increasing evidence could prove that locally derived estrogen contributes to the development of endometriotic lesions. Recently, high intra-tissue estrogen concentrations in endometriotic lesions have been described, suggesting local high concentration of estrogen was synthesized in endometriosis (Huhtinen K, Desai R, Stahlee M, et al. Endometrial and Endometriotic Concentrations of Estrone and Estradiol Are Determined by Local Metabolism Rather than Circulating Levels[J]. The Journal of Clinical Endocrinology & Metabolism, 2012, 97(11):4228-4235.DOI: 10.1210/jc.2012-1154). Accordingly, inhibition of local E2 production in the endometriotic lesion is regarded as a highly attractive mechanism of action for the treatment of endometriosis. AKR1C3 is expressed in large amounts in the endometriotic lesions and only marginally detectable in the ovary (Tina Šmuc, Hevir N, Martina RibičPucelj, et al. Disturbed estrogen and progesterone action in ovarian endometriosis[J]. Molecular & Cellular Endocrinology, 2009, 301(1):59-64.DOI: 10.1016/j.mce.2008.07.020). In the synergy action with CYP19A1 (aromatase), AKR1C3 is expected to be a key enzyme in local E2 production in endometriotic lesions, thereby generating a pro-estrogenic environment and stimulating the proliferation of the estrogen-sensitive endometriotic cells. The inhibition of AKR1C3 should therefore result in decreased local intra-tissue E2 levels and thereby decreasing the proliferation of endometriotic lesions. Effects on ovarian estrogen production are not expected, since AKR1C3 is only marginally expressed in the ovary and 17βHSD1 is the dominant ovarian hydroxysteroid dehydrogenase.

AKR1C3 is also a PGF2α synthase and beside the upregulation of AKR1C3 in endometriotic lesions, it has been shown that the levels of PGF2α were significantly higher in both the eutopic and ectopic endometria derived from women with peritoneal endometriosis than in similar tissues derived from women with ovarian endometrioma (Maša Sinreih, Anko M, Kene N H, et al. Expression of AKR1B1, AKR1C3 and other genes of prostaglandin F2α biosynthesis and action in ovarian endometriosis tissue and in model cell lines[J]. Chemico-Biological Interactions, 2015, 234(5-6):320-331.DOI: 10.1016/j.cbi.2014.11.009). PGF2α in endometriotic tissues is expected to contribute to inflammation, pain and proliferation in endometriosis patients and AKR1C3, expressed in endometriotic lesions, is expected to contribute to high local PGF2a levels in endometriotic tissues. AKR1C3 inhibition has the potential to relieve proliferation, pain and inflammation in endometriosis patients by locally reducing E2, testosterone and PGF2α levels in the endometriotic tissues.

AKR1C3 is closely related to polycystic ovary syndrome (PCOS). AKR1C3 plays a role in several pathologic conditions/diseases, which include polycystic ovary syndrome (PCOS).

PCOS is a common endocrine disorder, which affecting up to 10% of women of reproductive age. It is associated clinically with anovulatory infertility, dysfunctional bleeding, androgen excess, hyperinsulinemia and insulin resistance, obesity and metabolic syndrome (Fang Y. Insulin resistance and the polycystic ovary syndrome: Mechanism and implications for pathogenesis[J]. Endocrine Reviews, 1998, 18(6):774-800.DOI: 10.1210/edrv.18.6.0318). Four cardinal features of PCOS have been recognized by the Androgen Excess Society: ovulatory and menstrual dysfunction, biochemical hyperandrogenaemia, clinical hyperandrogonism (e.g. acne and hirsutism) and polycystic ovaries (Azziz R, Carmina E, Dewailly D, Diamanti-Kandarakis E, Escobar-Morreale HF, Futterweit W, et al. Position statement: criteria for defining polycystic ovary syndrome as a predominantly hyperandrogenic syndrome: an Androgen Excess Society.[J].Clin Endocrinol Metab 2006; 91:4237-45.DOI: 10.1055/s-2003-43304). The vast majority of women with PCOS will have the clinical symptoms of hyperandrogonism, e.g. acne, hirsutism, or anovulation manifest by primary subfertility or oligomenorrhea (LegroRichard S, Brzyski Robert G, Diamond Michae P, et.al, Letrozole versus Clomiphene for Infertility in the Polycystic Ovary Syndrome[J]. New England Journal of Medicine, 2014, 371(2):119-129.DOI: 10.1056/nejmoa1313517). Women with PCOS are prone to glucose intolerance and metabolic syndrome (Taponen S, Martikainen H, Jarvelin M R. Metabolic Cardiovascular Disease Risk Factors in Women With Self-Reported Symptoms of Oligomenorrhea and/or Hirsutism: Northern Finland Birth Cohort 1966 Study[J]. Journal of Clinical Endocrinology & Metabolism, 2004, 89(5):2114-8.DOI: 10.1210/jc.2003-031720), and with associated risk factors for cardiovascular disease and a likely increased risk in future cardiovascular events (Mani H, Levy MJ, Davies MJ, et al. Diabetes and cardiovascular events in women with polycystic ovary syndrome; a 20-year retrospective cohort study[J].Clin Endocrinol, 2013, 78(6):926-934.DOI: 10.1111/cen.12068). Hyperandrogonism, hirsutism and/or hyperandrogenaemia are the key components of the syndrome and are mandatory for the diagnosis of PCOS (Azziz R, Carmina E, Dewailly D, Diamanti-Kandarakis E, Escobar-Morreale HF, Futterweit W, et al. Position statement: criteria for defining polycystic ovary syndrome as a predominantly hyperandrogenic syndrome: an Androgen Excess Society.[J].Clin Endocrinol Metab 2006; 91:4237-45.DOI: 10.1055/s-2003-43304). While serum testosterone is a key factor for biochemical assessment of hyperandrogenaemia, androstenedione was suggested as a more reliable marker for PCOS-related androgen excess recently because it is circulating at high concentrations in women with PCOS (O'Reilly, Michael W, Taylor A E, Crabtree N J, et al. Hyperandrogenemia predicts metabolic phenotype in polycystic ovary syndrome: the utility of serum androstenedione[J]. The Journal of Clinical Endocrinology & Metabolism, 2014:jc.2013-3399.DOI: 10.1210/jc.2013-3399). PCOS has traditionally been regarded as a disorder of the ovary (Franks S, Gharani N, Gilling-Smith C.Polycystic ovary syndrome: evidence for a primary disorder of ovarian steroidogenesis[J]. Journal of Steroid Biochemistry and Molecular Biology, 1999, 69:269-272.DOI: 10.1016/s0960-0760(99)00044-8). However, the increased focus on the formation of extra-ovarian and extra-adrenal androgen in PCOS patients has highlighted the role of peripheral tissues such as adipose androgen formation (Quinkler M, Sinha B, Tomlinson JW, Bujalska IJ, Stewart PM, Arlt W(2004) Androgen generation in adipose tissue in women with simple obesity a site-specific role for 17{beta}hydroxysteroid dehydrogenase type 5. [J].J Endocrinol, 2004, 183:331-342.DOI: 10.1677/joe.1.05762). AKR1C3 is an androgen-activating enzyme and is known to predominantly convert androstenedione to testosterone. The upregulation of AKR1C3 in adipose tissue of PCOS patients has been described, indicating that ARK1C3 expression in adipose significantly contributed to the androgen formation for androstenedione in PCOS patients. In addition, it has been shown that AKR1C3 expression in adipocytes is significantly increased by insulin, indicating that the high concentration of insulin in PCOS patients is able to drive adipose androgen formation by increasing AKR1C3 activity in female subcutaneous adipose tissue (O"Reilly M, Gathercole L, Capper F, et al. Effect of insulin on AKR1C3 expression in female adipose tissue: in-vivo and in-vitro study of adipose androgen generation in polycystic ovary syndrome[J].The Lancet, 2015, 385:S16.DOI: 10.1016/S0140-6736(15)60331-2). AKR1C3 is also a PGF2α synthase and plays a suppressive role in the formation of endogenous ligands for the peroxisome proliferator-activated receptor γ (PPARgamma), which is a target for insulin-sensitizing drugs (Spiegelman et.al, Diabetes 1998, 47:507-514.DOI:10.2337/diabetes.47.4.507). Selective AKR1C3 inhibition may offer a novel therapeutic target to reduce androgen burden and improve the metabolic phenotype in PCOS (O"Reilly M, Gathercole L, Capper F, et al. Effect of insulin on AKR1C3 expression in female adipose tissue: in-vivo and in-vitro study of adipose androgen generation in polycystic ovary syndrome[J].The Lancet, 2015, 385:S16.DOI: 10.1016/S0140-6736(15)60331-2; Du et.al, J Clin Endocrinol Metab. 2009, 94(7):2594-2601.DOI:10.1210/jc.2009-0139).

AKR1C3 is closely related to cancer. AKR1C3 plays a role in several pathologic conditions/diseases, which include cancer.

AKR1C3 is overexpressed in numerous cancers, which includes those cancers of the prostate, breast, uterine, blood, lung, brain and kidney, such as endometrial carcinoma (Rizner TL, Smuc T, Rupreht R, Sinkovec J, Penning TM AKR1C1 and AKR1C3 may determine progesterone and estrogen ratios in endometrial cancer.[J]. Molecular & Cellular Endocrinology, 2005, 248(1-2):126-135.DOI: 10.1016/j.mce.2005.10.009), lung carcinoma (Lan Q, Mumford JL, Shen M, Demarini DM, Bonner MR, He X, Yeager M, Welch R, Chanock S, Tian L, Chapman RS, Zheng T, Keohavong P, Caporaso N, Rothman N. Oxidative damage-related genes AKR1C3 and OGG1 modulate risks for lung cancer due to exposure to PAH-rich coal combustion emissions. Carcinogenesis. 2004, 25 (11):2177-2181.DOI: 10.1093/carcin/bgh240), non-Hodgkin lymphoma (Lan Q, Zheng T, Shen M, et al. Genetic polymorphisms in the oxidative stress pathway and susceptibility to non-Hodgkin lymphoma. Hum Genet. 2007; 121:161-168.DOI:10.1007/s00439-006-0288-9), bladder carcinoma (Figueroa J D, Núria Malats, Montserrat Garcia-Closas, et al. Bladder cancer risk and genetic variation in AKR1C3 and other metabolizing genes [J]. Carcinogenesis, 2008, 29(10):1955-62..DOI: 10.1093/carcin/bgnl63), chronic myeloid leukaemia (Birtwistle J, Hayden R E, Khanim F L, et al. The aldo-keto reductase AKR1C3 contributes to 7, 12-dimethylbenz(a)anthracene-3, 4-dihydrodiol mediated oxidative DNA damage in myeloid cells: Implications for leukemogenesis[J]. Mutation Research, 2009, 662(1-2):67-74.DOI:10.1016/j.mrfmmm.2008.12.010), renal cell carcinoma Azzarello J T, Lin H K, Gherezghiher A, et al. Expression of AKR1C3 in renal cell carcinoma, papillary urothelial carcinoma, and Wilms' tumor[J]. International Journal of Clinical & Experimental Pathology, 2010, 3(2):147.PMID:20126582), and breast cancer (Byrns M C, Duan L, Lee S H, et al. Aldo-keto reductase 1C3 expression in MCF-7 cells reveals roles in steroid hormone and prostaglandin metabolism that may explain its over-expression in breast cancer[J]. J Steroid Biochem Mol Biol, 2010, 118(3):0-187.DOI:10.1016/j.jsbmb.2009.12.009). However, its upregulation frequently correlates with tumor invasiveness and aggressiveness (Azzarello J T, Lin H K, Gherezghiher A, et al. Expression of AKR1C3 in renal cell carcinoma, papillary urothelial carcinoma, and Wilms' tumor[J]. International Journal of Clinical & Experimental Pathology, 2010, 3(2):147.PMID:20126582; Birtwistle J, Hayden R E, Khanim F L, et al. The aldo-keto reductase AKR1C3 contributes to 7, 12-dimethylbenz(a)anthracene-3, 4-dihydrodiol mediated oxidative DNA damage in myeloid cells: Implications for leukemogenesis[J]. Mutation Research, 2009, 662(1-2):67-74.DOI:10.1016/j.mrfmmm.2008.12.010; Miller et.al, Aldo-keto reductase family 1 member C3 (AKR1C3) is expressed in adenocarcinoma and squamous cell carcinoma but not small cell carcinoma. [J].Int. J. Clin. Exp. Path. 2012, 5:278-289.PMID: 22670171). AKR1C3 is able to directly reduce estrone and progesterone to 17β-estradiol and 20α-hydroxyprogesterone, respectively, thereby enhancing this pro-proliferative signal (Smuc, Rizner, Expression of 17β-hydroxysteroid dehydrogenases and other estrogen-metabolizing enzymes in different cancer cell lines[J].Chem Biol Interact. 2009, 178:228-33.DOI:10.1016/j.cbi.2008.10.038).

Additionally, the prostaglandin F synthase activity of AKR1C3 catalyses the conversion of PGH2 to PGF2α and PGD2 to 11β-PGF2α, both are known to stimulate inflammation and proliferation. In the absence of AKR1C3 activity, PGD2 (instead of being converted to PGF2), spontaneously dehydrates and rearranges to form antiproliferative and anti-inflammatory PGJ2 isomers, including 15d-PGJ2.

In summary, AKR1C3 increases the proliferative PGF2 isomers and decreases the antiproliferative PGJ2 products, and therefore AKR1C3 has the potential to impact both hormone-dependent and hormoneindependent cancers. In breast cancer, it is postulated that under the action of AKR1C3, it can produce prostaglandin F2α (PTGFR) ligands whose activation results in carcinoma cell survival (Yoda T et.al, 11β-Prostaglandin F2α, a bioactive metabolite catalyzed by AKR1C3, stimulates prostaglandin F receptor and induces slug expression in breast cancer[J].Mol Cell Endocrinol. 15; 413:236-247.DOI:10.1016/j.mce.2015.07.008).

AKR1C3 plays a role in several pathologic conditions/diseases, which include prostate cancer. Increased expression of AKR1C3 has been associated with prostate cancer progression and aggressiveness (Stanbrough M et.al, Increased expression of genes converting in androgen-independent prostate cancer[J].Cancer Res 2006, 66:2815-25.DOI:10.1158/0008-5472.can-05-4000; Wako K, Kawasaki T, Yamana K. et al. Expression of androgen receptor through androgen-converting enzymes is associated with biological aggressiveness in prostate cancer. J Clin Pathol. 2008 Apr; 61(4):448-54.DOI: 10.1136/jcp.2007.050906). In hormone-dependent prostate cancer, AKR1C3 converts androstenedione to testosterone, which, in turn, excessively activates androgen receptors and promotes tumor growth (Penning T M, Steckelbroeck S, Bauman D R, et al. Aldo-keto reductase (AKR) 1C3: role in prostate disease and the development of specific inhibitors.[J]. Molecular & Cellular Endocrinology, 2006, 248(1):182-191.DOI:10.1016/j.mce.2005.12.009). In castration-resistant prostate cancer (CRPC), AKR1C3 is involved in intratumoral androgen biosynthesis, and it facilitates the conversion of weak androgens androstenedione (A' dione) and 5α-androstanedione (5α-dione) to the more active androgens testosterone and DHT, respectively (Liu C, Lou W, Zhu Y, et al. Intracrine Androgens and AKR1C3 Activation Confer Resistance to Enzalutamide in Prostate Cancer[J]. Cancer Research, 2015, 75(7):1413-1422.DOI:10.1158/0008-5472.can-14-3080; Fung, K-M. Increased expression of type 2 3-hydroxysteroid dehydrogenase/type 5 17-hydroxysteroid dehydrogenase (AKR1C3) and its relationship with androgen receptor in prostate carcinoma[J]. Endocrine Related Cancer, 2006, 13(1):169-180.DOI:10.1677/erc.1.01048). Importantly, AKR1C3 expression has been shown to be increased in patients with CRPC compared with in patients with primary prostate cancer (Stanbrough et.al, Cancer Res 2006, 66: 2815-2825.DOI:10.1158/0008-5472.CAN-05-4000; Hamid AR, Pfeiffer MJ, Verhaegh GW, Schaafsma E, Brandt A, Sweep FC, Sedelaar JP, Schalken JA. Aldo-keto reductase family 1 member C3 (AKR1C3) is a biomarker and therapeutic target for castration-resistant prostate cancer. Mol. Med. 2012; 18:1449-1455.DOI: 10.2119/molmed.2012.00296; Pfeiffer MJ, Smit FP, Sedelaar JP et al (2011) Steroidogenic enzymes and stem cell markers are upregulated during androgen deprivation in prostate cancer. Mol Med 17:657-664. D01:10.2119/molmed.2010.00143). A genetic polymorphism in the AKR1C3 gene coding for AKR1C3 was also shown to be an independent predictor of prostate cancer (Yu et.al, PLoS One 2013, 8(1):e54627.DOI: 10.1371/journal.pone.0054627). Moreover, AKR1C3-dependent androgen de novo synthesis was suggested to be a potential mechanism of resistance to CYP17A1 inhibitors, such as abiraterone (Mostaghel et.al, Clin Cancer Res 2011, 17:5913-5925.DOI:10.1158/1078-0432.CCR-11-0728; Cai et.al, Cancer Res 2011, 71:6503-6513.DOI:10.1158/0008-5472.CAN-11-0532). Therefore, AKR1C3 may be a promising therapeutic target in patients with CRPC (Adeniji et.al, J Steroid Biochem Mol Biol 2013, 137:136-149.DOI:I0.I021/jm3017656). An AKR1C3 inhibitor was tested in patients with metastatic castration-resistant prostate cancer in a multicentre phase I/II study. However, the novel androgen biosynthesis inhibitor showed no relevant evidence of clinical activity (Loriot et.al, Invest New Drugs 2014, 32:995-1004.DOI:10.1007/s10637-014-0101-x). Recent information shows that AKR1C3 activation in CRPC is a critical resistance mechanism associated with antiandrogen (enzalutamide) resistance. It could be shown that androgen precursors such as cholesterol, DHEA and progesterone, as well as androgens are highly upregulated in enzalutamide-resistant prostate cancer cells compared to the parental cells. The information shows that the inhibition pathways of AKR1C3 could act as an enzalutamide-sensitizing treatment and have a restorative efficacy in patients with enzalutamide-resistant CRPC (Liu C, Lou W, Zhu Y, et al. Intracrine Androgens and AKR1C3 Activation Confer Resistance to Enzalutamide in Prostate Cancer[J]. Cancer Research, 2015, 75(7):1413-1422.DOI:10.1158/0008-5472.can-14-3080). It is postulated that the co-treatment with an AKR1C3 inhibitor will overcome the resistance to enzalutamide and improve survival of advanced prostate cancer patients (Thoma et.al, Nature Reviews Urology 2015, 12:124.DOI:10.1038/nrurol.2015.23).

AKR1C3 plays a role in several pathologic conditions/diseases, which include anthracvcline resistant cancer. Anthracyclines (or anthracycline antibiotics) are a class of drugs which are used in cancer chemotherapy and derived from Streptomyces bacterium Streptomyces peucetius var. caesius (Fujiwara et.al, Streptomyces bacterium Streptomyces peucetius var. Caesius, Critical Reviews in Biotechnology, 1985, 3 (2):133.DOI: 10.3109/07388558509150782). These compounds are used to treat many cancers, including leukaemia, lymphoma, breast cancer, stomach cancer, uterine cancer, ovarian cancer, bladder cancer and lung cancer. The anthracyclines are the most effective anticancer treatments ever developed. However, the clinical success of anthracyclines for cancer treatment is overshadowed by drug resistance. It has become widely accepted that the elevated enzymatic reduction of anthracyclines to their less potent secondary C13-hydroxy metabolites constitutes one of the mechanisms that cause anthracycline resistance in tumors (Gavelova et.al, 2008 Chem. Biol. Interact 176, 9-18.DOI: 10.1016/j.cbi.2008.07.011; Heibein et.al, 2012 BMC Cancer 12, 381.DOI:10.1186/1471-2407-12-381). The enzymatic metabolism of doxorubicin is responsible for the cardiomyopathy observed upon doxorubicin chemotherapy. The existing literature shows that AKR1C3 was involved in the metabolism of clinically administered anthracyclines such as doxorubicin and daunorubicin (Novotna et.al, Toxicol. Letter 2008, 181:1-6.DOI:10.1016/j.toxlet.2008.06.8586). In 2012, a correlation of an AKR1C3 genetic variant with doxorubicin pharmacodynamics has been shown in Asian breast cancer patients: one genetic variant was associated with longer progression-free survival and overall survival after doxorubicin-based therapy, which suggesting the potential interaction with the doxorubicin metabolism (Voon et.al, British J of Clin Pharmacology 2012, 75:1497-1505.DOI:10.1111/bcp.12021). Recently, it could be demonstrated that AKR1C3 contributes to the resistance of cancer cells to anthracycline treatment and therefore concomitant administration of a specific AKR1C3 inhibitor with anthracyclines could be an efficient strategy for the successful prevention and treatment of anthracycline resistant tumors (Hofman et.al, Toxicology and Applied Pharmacology 2014, 278:238-248.DOI:10.1016/j.taap.2014.04.027).

Studies have also shown that AKR1C3 plays a key role in radiotherapy resistance of esophageal cancer, prostate cancer and non-small cell lung cancer (Sun et al Overexpression of AKR1C3 significantly enhances human prostate cancer cells resistance to radiation. Oncotarget. 2016 Jul 26; 7(30):48050-48058. DOI: 10.18632/oncotarget.10347; Xiong et al.; Elevated Expression of AKR1C3 Increases Resistance of Cancer Cells to Ionizing Radiation via Modulation of Oxidative Stress. PLoS ONE 2014 9(11): e111911. DOI:10.1371/journal.pone.01 11911). The anti-radiation effect of AKR1C3 in prostate cancer cells is studied by using the prostate cell line DU145 and its corresponding cell line AKR1C3-over which stably and highly expresses AKR1C3. Indomethacin is a specific inhibitor for AKR1C3, and it can obviously enhance the sensitivity of prostate cells to radiotherapy. The research found that PGF2α can not only promote the proliferation of prostate cancer cells, but also enhance the resistance of prostate cancer cells to radiation. The accumulation of PGF2α in AKR1C3-over cells leads to activation of MAPK pathway and inhibition of PPARγ expression. At the same time, the research found that the high expression of AKR1C3 can cause radiation resistance of NSCL (Xie et al; Aldo-keto reductase 1C3 may be a new radioresistance marker in non-small-cell lung cancer. Cancer Gene Ther. 2013 Apr; 20(4):260-6. DOI:10.1038/cgt.2013.15). In A549/R and SPCA1/R cells, AKR1C3 mRNA and protein levels are significantly upregulated. The AKR1C3 protein levels in A549/R and SPCA1/R are 6.2 times and 3.5 times higher than those in sensitive control cells, respectively. The above research shows that reducing the expression of AKR1C3 can effectively increase the sensitivity of NSCLC cells to radiotherapy.

The expression of AKR1C3 is significantly upregulated in patients of anti-immunotherapy. It has been reported in the literature that among 13 patients with renal cell carcinoma who received anti-PD-1 treatment, there were 4 responders (R) and 9 nonresponders (NR), and genome-wide analysis showed that there were significant differences in the expression of 234 genes between R and NR. One of the significant difference is that the expression of AKR1C3 is significantly increased in nonresponders (Ascierto 2015 The intratumoral balance between metabolic and immunologic gene expression is associated with anti-PD-1 response in patients with renal cell carcinoma Cancer Immunol Res 2016 4 726-733, doi:10.1158/2326-6066.CIR-16-0072).

The research shows that the combination of an AKR1C3 specific inhibitor and chemotherapy drugs such as daunorubicin or cytarabine can greatly increase the killing ability to blood cancer cells (Verma et al, Potent and Highly Selective Aldo-Keto Reductase 1C3 (AKR1C3) Inhibitors Act as Chemotherapeutic Potentiators in Acute Myeloid Leukemia and T-Cell Acute Lymphoblastic Leukemia. J. Med. Chem. 2019, 62, 7: 3590-3616, DOI: 10.1021/acs.jmedchem.9b00090).

AKR1C3 plays a role in several pathologic conditions/diseases, which include atopic dermatitis. The attack of antigen on atopic individuals causes the release of PGD2 and histamine, which shows that PGD2 hardly triggers the immediate hypersensitivity reactions of human skin and that PGD2 is a lipid mediator that promotes skin inflammation in atopic dermatitis (AD) (Barr et.al, Br J Pharmacol. 1988, 94:773-80.PMID: 2460180; Satoh et.al, J Immunol. 2006, 177:2621-9.DOI:10.4049/jimmunol.177.4.2621; Shimura et.al, Am J Pathol. 2010, 176:227-37.DOI:10.2353/ajpath.2010.090111). PGD2 is a relatively unstable pro-inflammatory mediator, and it spontaneously converts to the potent anti-inflammatory mediator 15d-PGJ2. That conversion is diverted by the metabolism of PGD2 to the pro-inflammatory 9α, 11β-PGF2 by AKR1C3 (Mantel et.al, Exp Dermatol. 2016, 25(1):38-43.DOI:10.1111/exd.12854). It was demonstrated that AKR1C3 is upregulated in human AD samples and it was postulated that AKR1C3 plays a role in mediating inflammation in skin pathology, especially atopic dermatitis and in keloids (Mantel et.al, J Invest Dermatol 2012, 132(4):1103-1110.DOI:10.1038/jid.2011.412; Mantel et.al, Exp Dermatol. 2016, 25(1):38-43.DOI:10.1111/exd.12854). AKR1C3 inhibition might be a novel option for the treatment of AD and keloids.

AKR1C3 plays a role in several pathologic conditions/diseases, which include inflammation. AKR1C3 is involved in prostaglandin biosynthesis, and it catalyzes the conversion of PGH2 to PGF2α and PGD2 to 11β-PGF2. It has been postulated that the expression and upregulation of AKR1C3 supports inflammation by directly causing an increase in 9α, 11β-PGF2 synthesis rates and diverting the spontaneous generation of the potent anti-inflammatory mediator 15d-PGJ2 (Mantel et.al, J Invest Dermatol 2012, 132(4):1103-1110.DOI:10.1038/jid.2011.412). This function of AKR1C3 is also involved in HL-60 cells (Desmond et.al, Cancer Res 2003, 63:505-512.PubMed:12543809) and in MCF-7 cells (Byrns et.al, J Steroid Biochem Mol Biol 2010, 118:177-187.DOI:10.1016/j.jsbmb.2009.12.009). The inhibition of AKR1C3 is postulated to increase 15d-PGJ2 (an anti-inflammatory lipid), which mostly mediates its actions directly via activation of peroxisome proliferator-activated receptor γ (PPAR-γ) and/or inhibition of NF-KB signaling in immune cells (Maggi et.al, Diabetes 2000, 49:346-355.DOI:10.2337/diabetes.49.3.346; Scher et.al, Clinical Immunology 2005, 114:100-109.DOL10.1016/J.dim.2004.09.008). Previous information has shown that PPAR-γ activation attenuates allergen-induced inflammation in skin and lungs of mice (Ward et.al, Carcinogenesis. 2006, 27(5):1074-80.DOI: 10.1093/carcin/bgi329; Dahten et.al, J Invest Dermatol. 2008, 128(9):2211-8.DOI:10.1038/jid.2008.84). This suggests a role for AKR1C3 inhibition in suppressing of inflammation.

AKR1C3 plays a role in several pathologic conditions/diseases, which include further diseases. Furthermore, AKR1C3 inhibitors have potential for the treatment of the following conditions/diseases: prostatic hyperplasia (Roberts et.al, Prostate 2006, 66 (4), 392-404.DOI:10.1002/pros.20362), alopecia (L.Colombe et.al, Exp Dermatol 2007, 16 (9), 762-769.DOI:10.1111/j.1600-0625.2007.00639.x), obesity (P.A.Svensson et.al, Cell Mol Biol Lett 2008, 13 (4), 599-613.DOI:100.2478/s11658-008-0025-6), premature sexual maturation (C. He, Hum Genet 2010, 128 (5), 515-527.DOI:10.1007/s00439-010-0878-4.) and chronic obstructive pulmonary disease (S. Pierrou, Am J Respir Crit Care 2007, 175 (6), 577-586.DOI:10.1164/rccm.200607-931OC).

It was surprisingly found that the compounds of the present invention have the effects of inhibiting the activity of AKR1C3, and may therefore be used for the treatment or prophylaxis of AKR1C3 related disorders such as gynecological disorders (in particular, endometriosis-related and polycystic ovary syndrome-related gynecological disorders), conditions and diseases; metabolic disorders; hyperproliferative disorders, conditions and diseases; inflammation disorders; and the other related diseases or disorders.

Based on this, the following technical solutions relating to pharmaceutical use are also provided.

A medicament containing the above compound, and the pharmaceutically acceptable salt, or the solvate, or the isotopically substituted compound thereof.

The medicament is used for preventing or treating diseases related to an AKR1C3 inhibitor.

In a further embodiment, the medicament is used for prevention or treatment of diseases/conditions comprising endometriosis, uterine leiomyoma, uterine hemorrhagic disorders, dysmenorrhea, prostatic hyperplasia, acne, seborrhea, alopecia, premature sexual maturation, polycystic ovary syndrome, chronic obstructive pulmonary disease COPD, obesity, inflammatory pain, cancer, inflammation or cancer pain.

In a still further embodiment, the cancer comprises prostate cancer, primary prostate cancer, advanced prostate cancer, metastatic prostate cancer, hormone naive prostate cancer, refractory prostate cancer or castration-resistant prostate cancer CRPC, breast cancer, lung cancer, endometrial cancer, renal cell carcinoma, bladder cancer, non-Hodgkin lymphoma, and acute myeloid leukemia AML, T-cell acute lymphoblastic leukemia TALL, and leukemia.

Use of the above compound, and the pharmaceutically acceptable salt, or the solvate, or the isotopically substituted compound thereof, in the manufacture of a medicament for treating or preventing a relevant disease/disorder, wherein the disease/disorder comprises endometriosis, uterine leiomyoma, uterine hemorrhagic disorders, dysmenorrhea, prostatic hyperplasia, acne, seborrhea, alopecia, premature sexual maturation, polycystic ovary syndrome, chronic obstructive pulmonary disease COPD, obesity, inflammatory pain, cancer, inflammation or cancer pain.

The present application further provides a medicament for enhancing sensitivity to radiation therapy for cancers or tumors, wherein the medicament comprises the above compound, and the pharmaceutically acceptable salt, or the solvate, or the isotopically substituted compound thereof, wherein the medicament is used for increasing the efficacy of radiation therapy when a patient with a cancer or a tumor is resistant to radiation therapy, or treating a patient with a cancer or a tumor who is resistant to radiation therapy.

The present application further provides a medicament for enhancing sensitivity to immunotherapy for cancers or tumors, wherein the medicament comprises the above compound, and the pharmaceutically acceptable salt, or the solvate, or the isotopically substituted compound thereof, wherein the medicament is used for increasing the efficacy of immunotherapy when a patient with a cancer or a tumor is resistant to immunotherapy, or treating a patient with a cancer or a tumor who is resistant to immunotherapy.

The present application further provides a medicament for enhancing sensitivity to chemotherapy for cancers or tumors, wherein the medicament comprises the above compound, and the pharmaceutically acceptable salt, or the solvate, or the isotopically substituted compound thereof, wherein the medicament is used for increasing the efficacy of chemotherapy when a patient with a cancer or a tumor is resistant to chemotherapy, or treating a patient with a cancer or a tumor who is resistant to chemotherapy.

The present application further provides the use of the above compound, and the pharmaceutically acceptable salt, or the solvate, or the isotopically substituted compound thereof, in the manufacture of a medicament for enhancing the efficacy of radiation therapy when a patient with a cancer or a tumor is resistant to immunotherapy, or for treating a patient with a cancer or a tumor who is resistant to immunotherapy.

The present application further provides a medicament for enhancing sensitivity to chemotherapy for a cancer or a tumor comprising the above compound, and the pharmaceutically acceptable salt, or the solvate, or the isotopically substituted compound thereof, wherein the medicament is used for increasing the efficacy of chemotherapy when a patient with a cancer or a tumor is resistant to chemotherapy, or treating a patient with a cancer or a tumor who is resistant to chemotherapy, and wherein the medicament used in the chemotherapy contains daunorubicin or cytarabine.

The present application further provides use of the above compound, and the pharmaceutically acceptable salt, or the solvate, or the isotopically substituted compound thereof, for enhancing the sensitivity to radiation therapy for a cancer or a tumor, or for enhancing the sensitivity to the chemotherapy for a cancer or a tumor using a medicament containing daunorubicin or cytarabine.

The present application further provides use of the above compound, and the pharmaceutically acceptable salt, or the solvate, or the isotopically substituted compound thereof, as an AKR1C3 enzyme inhibitor.

The present application further provides use of the above compound, and the pharmaceutically acceptable salt, or the solvate, or the isotopically substituted compound thereof in the manufacture of a medicament, wherein the medicament is an AKR1C3 enzyme inhibitor.

The present application further provides use of the above compound, and the pharmaceutically acceptable salt, or the solvate, or the isotopically substituted compound thereof, in the manufacture of a medicament used for enhancing the efficacy of radiation therapy when a patient with a cancer or a tumor is resistant to radiation therapy, or for treating a patient with a cancer or a tumor who is resistant to radiation therapy.

The present application further provides use of the above compound, and the pharmaceutically acceptable salt, or the solvate, or the isotopically substituted compound thereof, in the manufacture of a medicament used for enhancing the efficacy of chemotherapy when a patient with a cancer or a tumor is to chemotherapy, or for treating a patient with a cancer or a tumor who is resistant to chemotherapy.

The present application further provides use of the above compound, and the pharmaceutically acceptable salt, or the solvate, or the isotopically substituted compound thereof, in the manufacture of a medicament used for enhancing the efficacy of a chemotherapy when a patient with a cancer or a tumor is to chemotherapy, or for treating a patient with a cancer or a tumor who is resistant to chemotherapy, wherein the medicament used in the chemotherapy contains daunorubicin or cytarabine.

With respect to the medicament or formulation described herein, the prepared medicament comprises a particular dosage range of the indicated compound, or a salt or a solvate thereof, and/or the prepared medicament is in a particular dosage form and is administered using a specific mode of administration.

With respect to the uses described herein, the medicament prepared may further comprise a pharmaceutically acceptable adjuvant or excipient. The medicament may be in any dosage form for clinical administration, such as tablets, suppositories, dispersible tablets, enteric tablets, chewable tablets, orally disintegrating tablets, capsules, dragees, granules, dry powders, oral solutions, small needles for injection, lyophilized powder for injection, or infusion solutions. Depending on the particular dosage form and mode of administration, the pharmaceutically acceptable excipient or excipient in the medicament may include one or more of diluents, solubilizers, disintegrants, suspending agents, lubricants, binders, fillers, flavoring agents, sweetening agents, antioxidants, surfactants, preservatives, wrapping agents, pigments, and the like.

Preferably, the patient is a mammal, more preferably a human.

### DESCRIPTION OF EMBODIMENTS

The present application will be described below with reference to specific examples. It will be appreciated by those skilled in the art that these examples are intended to describe the present application only and are not intended to limit the scope of the present application in any way.

The experimental methods in the following examples, unless otherwise specified, are all conventional methods. The raw materials of the medicaments, the reagent materials, and the like used in the following examples are all commercially available products unless otherwise specified.

"Patient" and "subject" are used interchangeably to refer to a mammal in need of treatment for cancer. Typically, the patient is a human. Typically, the patient is a human diagnosed with cancer. In certain embodiments, a "patient" or "subject" may refer to a non-human mammal, such as non-human primate, dog, cat, rabbit, pig, mouse or rat, used in screening, characterizing, and evaluating drugs and therapies.

"Prodrug" refers to a compound that, upon feeding or administration, is metabolized or otherwise converted to a biologically active or more active compound (or drug) with respect to at least one property. A prodrug is chemically modified relative to the drug in a manner that renders it less active or inactive relative to the drug, but the chemical modification is such that the corresponding drug is generated by metabolic or other biological processes following administration of the prodrug. A prodrug may have altered metabolic stability or transport characteristics, fewer side effects or lower toxicity, or improved flavor relative to the active drug (see, for example, reference Nogrady, 1985, Medicinal Chemistry A Biochemical Approach, Oxford University Press, New York, pages 388-392, which is incorporated herein by reference). A prodrug may be synthesized using reactants other than the corresponding drug.

"Solid tumor" refers to solid tumors including, but not limited to, metastatic tumors in bone, brain, liver, lung, lymph nodes, pancreas, prostate, skin, and soft tissue (sarcoma).

"Therapeutically effective amount" of a drug refers to an amount of a drug that, when fed or administered to a patient with cancer, will have the intended therapeutic effect, e.g., alleviation, amelioration, palliation or elimination of one or more clinical manifestations of the cancer in the patient. A therapeutic effect does not necessarily occur by feeding or administration of one dose, and may occur only after feeding or administration of a series of doses. Thus, a therapeutically effective amount may be fed or administered in one or more administrations.

"Treatment" of a condition or patient refers to taking steps to obtain beneficial or desired results including clinical results. For purposes of this application, beneficial or desired clinical results include, but are not limited to, alleviation or amelioration of one or more symptoms of a cancer; diminishment of extent of disease; delay or slowing of disease progression; amelioration, palliation, or stabilization of the disease state; or other beneficial results. In some cases, treatment of a cancer may result in partial response or stable disease.

"Tumor cell" refers to a tumor cell of any appropriate species (e.g., mammalian, such as murine, canine, feline, equine, or human).

Radiation therapy, i.e., radiation therapy of a cancer, has a variety of embodiments, and the most commonly used being:
Photon (γ-ray) beam emission, in which a number of photon (γ-ray) beams are emitted by a linear accelerator;
Neutron beam radiation, which can be used to treat certain carcinomas with narrow tissue margins;
Electron beam radiation, which is useful in the treatment of skin or superficial carcinomas due to its very shallow depth of penetrating tissues;
Proton radiation, which, although has limitation in use, provides a sharp ray perimeter for a very narrow field of illumination requiring a certain depth;
Brachytherapy, in which a powerful source of radiation is implanted through a needle into the tumor tissue itself (e.g., a prostate or a lung) to achieve an effect with a small range and a high dose;
Systemic radionuclide therapy, which can be used for organ receptors with nuclide uptake (such as thyroid cancer) or receptors that inhibit systemic skeletal sites (such as radioactive strontium to treat metastatic prostate cancer);
Curative radiation therapy, which generally entails inclusion of the tumor locally or its local area within the field of irradiation;
The damage of radioactive irradiation to cells is non-selective and non-specific and produces complex effects on DNA, with the superior efficacy depending on how much the damage to cells exceeds the ability thereof to repair. In general, a normal tissue is repaired more efficiently than cancer cells, so that radiation therapy can benefit the patients.

Chemotherapy, which is a short term for chemodrug therapy, is used to kill cancer cells using chemotherapeutic drugs for therapeutic purposes. Chemotherapy is currently one of the most effective means to treat cancers. Chemotherapy, surgery and radiotherapy together are referred to as the three major treatments for cancers. Surgery and radiotherapy are local treatments that are only effective against tumors at the site of treatment, and effective treatments are difficult to exert for potential metastatic lesions (cancer cells actually have metastasized, but not yet clinically discovered and detectable because of limitations of current technical means) and cancers that have already developed clinical metastases. Whereas chemotherapy is a means of systemic treatment, chemotherapy drugs circulate throughout the vast majority of organs and tissues throughout the body with the blood, whatever route of administration is used (oral, intravenous and body cavity administration, etc.). Therefore, chemotherapy is the primary treatment for some tumors that have a tendency to spread systemically as well as mid-late tumors that have metastasized. Chemotherapeutic drugs in general are cytotoxic drugs that kill cells directly, but due to certain different characteristics between cancer cells and normal cells, chemotherapeutic drugs often have a greater killing effect for cancer cells, which can benefit the patients. However, chemotherapy still suffers from serious toxic side effects, including digestive system reactions, bone marrow suppression, hair loss, and some of these toxic side effects may not continue to receive chemotherapy or some chemotherapeutic agent.

Regarding immunotherapy, normally, the body's immune system can recognize and clear tumor cells from the tumor microenvironment, but tumor cells, in order to survive and grow, can adopt different strategies such that the body's immune system is suppressed and unable to kill tumor cells properly, thereby surviving various stages of an anti-tumor immune response. The process by which tumor cells are recognized and cleared by the human immune system (tumor-immune cycle) has multiple stages, and any of the stages is inhibited, it will lead to the result that tumor cells cannot be cleared normally. Different tumors can inhibit the effective recognition and killing of tumor cells by the immune system through abnormalities in different stages and thus produce immune tolerance, and even the development of tumors can be promoted.

Tumor immunotherapy is a treatment that restores the body's normal anti-tumor immune response to control and clear tumors by reinitiating and maintaining the tumor-immune cycle. Tumor immunotherapy includes, among others, monoclonal antibody-based immune checkpoint inhibitors, therapeutic antibodies, cancer vaccines, cell therapies, and small molecule inhibitors. In recent years, there has been ongoing good news for tumor immunotherapy, and at present, it has already shown strong anti-tumor activity in the treatment of a variety of tumors such as melanoma, non-small cell lung cancer, renal cancer and prostate cancer, and a number of tumor immunotherapy drugs have been approved for clinical use.

The foregoing description of specific embodiments of the application does not limit the application, and those skilled in the art can make various changes and modifications in light of the application without departing from the spirit of the application, which shall fall within the scope of the appended claims.

The following is specific tests and examples of the present application.

The following tests will reveal the in vitro inhibitory activity of AKR1C3 inhibitors developed by the applicant, who hereby state that the right to the following experimental data belongs to the applicant.

The applicant states that some of the specific compounds disclosed in the tests below can be synthesized based on specific synthetic methods and synthetic routes of the compounds disclosed herein, with reference to patent publications (such as Application No. PCT/US2016/021581, Publication No. WO2016/145092; Application No. PCT/US2016/062114, Publication No. WO 2017/087428) or other publications (although the substrates are different and the yields are either high or low, but the technicians who have mastered the skills of organic synthesis in this field, such as pharmaceutical chemistry and organic chemistry, can still synthesize them), and the applicant has confirmed the structure by NMR and MS. This application provides synthesis methods, NMR data and in vitro inhibitory activities against AKR1C3 enzyme of representative compounds.

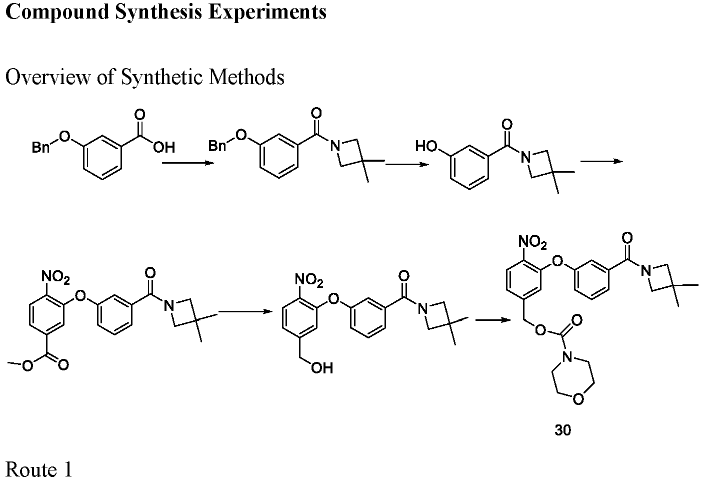

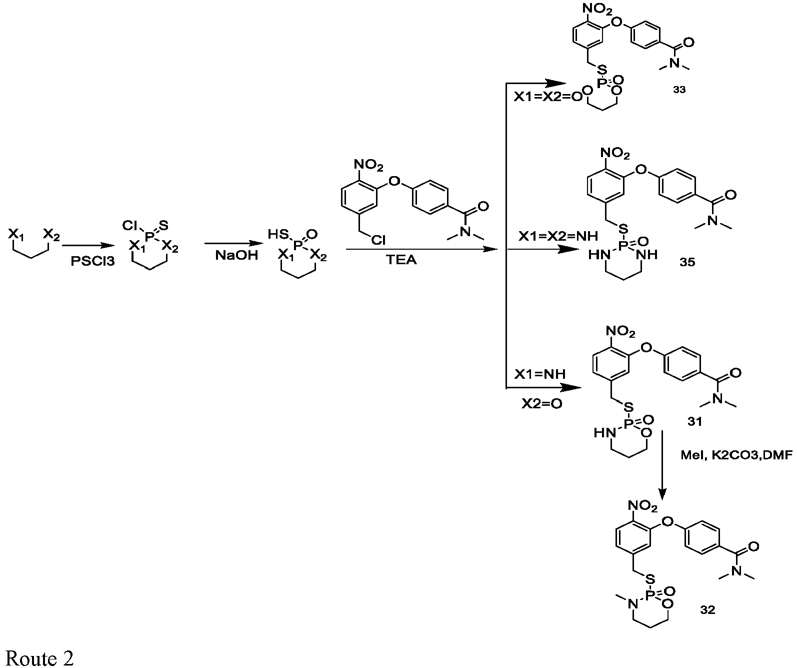

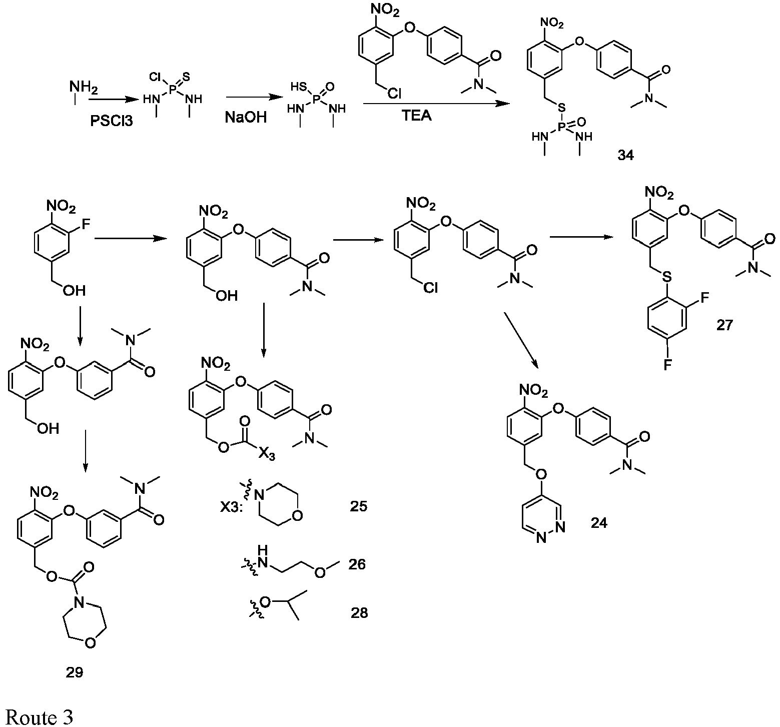

### Description of English Abbreviations

MTBE, methyl tert-butyl ether; DMAP, 4-dimethylaminopyridine; T₃P, propylphosphonic anhydride; THF, tetrahydrofuran; DCM, dichloromethane; EA or EtOAC, ethyl acetate; TEA, triethylamine; HPLC, high performance liquid chromatography; DBAD, di-tert-butyl azodicarboxylate; TFA, trifluoroacetic acid; LCMS, liquid chromatography - mass spectrometer; EtOH, ethanol; T-BuOH, tert-butanol; DMF, dimethylformamide; PE, petroleum ether; eq, equivalent i.e. molar ratio; TBAF, tetrabutylammonium fluoride; DIPEA, N, N-diisopropylethylamine; reflux, backflow; rt, room temperature.

The chemical reagents and drugs whose sources are not indicated in the synthesis process are analytically pure or chemically pure, and they are all purchased from commercial reagent companies.

Reference shall be made to the interpretations in the field of organic chemistry for other English abbreviations.

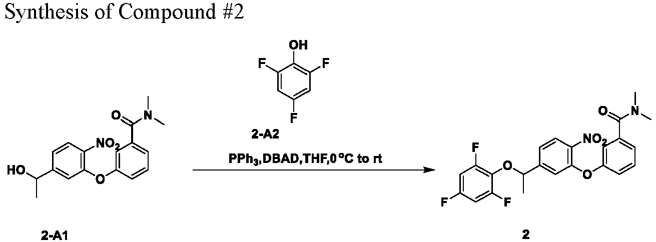

Under the protection of nitrogen, 2-A1 (500mg, 1.51 mmol, synthesized with reference to the synthesis method of Compound #29) and 2-A2 (336 mg, 2.27 mmol, commercially available) were dissolved in anhydrous THF (10 mL). The reactants were cooled to 0°C, triphenylphosphonium (991 mg, 3.78 mmol) was added, then a solution of di-tert-butyl azodicarboxylate (870 mg, 3.78 mmol) in THF (5 mL) was slowly added dropwise, with the temperature being kept at 0°C for half an hour. Then the reactants were stirred at room temperature for 2.5 hours. After the reaction was completed, water (5 mL) was added dropwise at 0°C. The reactants were extracted with DCM (10mL×3), washed with water (3mL×2), dried and concentrated, followed by high performance liquid chromatography to obtain pure Compound #2 (340 mg, 48.9%) as a pale yellow oil. ¹H-NMR(400M, CD₃OD): 88.01(d, *J*=8.4Hz, 1H), 7.52-7.47(m, 2H), 7.45(s, 2H), 7.30(d, *J*=1.2Hz, 1H), 7.25-7.23(m, 1H), 7.10-7.07(m, 2H), 5.54-5.52(m, 1H), 3.08(s, 3H), 2.98(s, 3H), 1.61(d, *J*=6.4Hz, 3H).

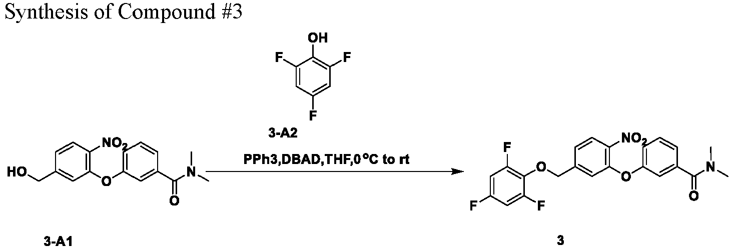

Under the protection of nitrogen, 3-A1 (200mg, 0.632mmol, synthesized with reference to the synthesis method of Compound #29) and 3-A2 (140mg, 0.948mmol, commercially available) were dissolved in anhydrous THF (5 mL). The reactants were cooled to 0 °C, triphenylphosphonium (414mg, 1.58mmol) was added, then a solution of di-tert-butyl azodicarboxylate (363mg, 1.58mmol) in THF (2 mL) was slowly added dropwise, with the temperature being kept at 0°C for half an hour. Then the reactants were stirred at room temperature for 2.5 hours. After the reaction was completed, water (5 mL) was added dropwise at 0°C. The reactants were extracted with DCM (5mL×3), washed with water (2mL×2), dried and concentrated, followed by high performance liquid chromatography to obtain pure Compound #3 (160 mg, with a yield of 56.7%) as a pale yellow oil. ¹H-NMR(400M, CDCl₃): 87.95(d, J=8.4Hz, 1H), 7.38-7.32(m, 2H), 7.25(s, 1H), 7.20-7.16(m, 3H), 7.05-7.02(m, 2H), 4.94(s, 2H), 3.02(s, 3H), 2.90(s, 3H).

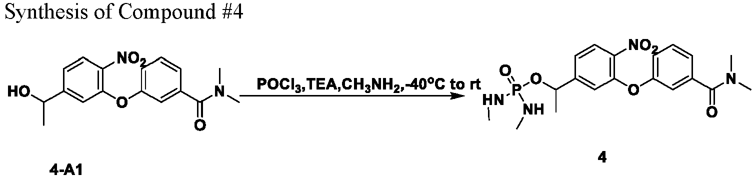

Under the protection of nitrogen, phosphorus oxychloride (580 mg, 3.78 mmol) was added dropwise to anhydrous DCM (5 mL). The reactants were cooled to -40°C, a solution of 4-A1 (500 mg, 1.51 mmol, synthesized with reference to the synthesis method of Compound #29) in DCM (3 mL) was added dropwise, then a solution of TEA (397 mg, 3.9 mmol) in DCM (2 mL) was added dropwise, with the temperature being kept at -40°C for 6h until the raw material disappeared. Methylamine (1.6 g, 12.8 mmol, 25% in THF) was added dropwise, then a solution of TEA (1.29 g, 12.85 mmol) in DCM (5 mL) was added dropwise. The temperature was kept at -40 °C for half an hour and then is naturally increased to an ambient temperature, and the reactants were stirred overnight. After the reaction was completed, the reactants were cooled to 0°C, a potassium carbonate solution (1g, 10 mL) was added dropwise. The reactants were extracted with DCM (10mL×3), washed with water (5mL×2), dried and concentrated, followed by high performance liquid chromatography to obtain the pure product (380mg, with a yield of 57.7%) as a pale yellow viscous oil. ¹H-NMR(400MHz, CDCl₃): 87.95(d, J=8.4Hz, 1H), 7.42(t, J=7.9Hz, 1H), 7.21-7.18(m, 2H), 7.12-7.07(m, 2H), 7.04(s, 1H), 5.47-5.44(m, 1H), 3.08(s, 3H), 2.97(s, 3H), 2.59(d,J=12.2Hz, 3H), 2.51(m, 2H), 2.38(d,J=12.2Hz, 3H), 1.53(d, *J*=6.5Hz, 3H). MS: Calculated 436.2, found 437.1([M+H]⁺).

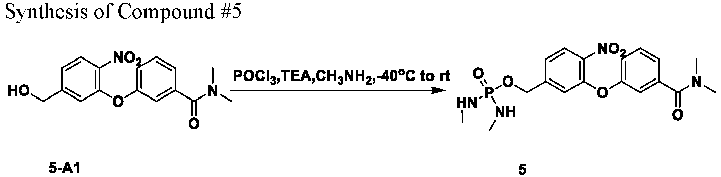

Under the protection of nitrogen, phosphorus oxychloride (242mg, 1.58mmol) was added dropwise to anhydrous DCM (5 mL). The reactants were cooled to -40°C, a solution of 5-A1 (200mg, 0.63mmol, synthesized with reference to the synthesis method of Compound #29) in DCM (3 mL) was added dropwise, then a solution of TEA (166mg, 1.64mmol) in DCM (2 mL) was added dropwise, with the temperature being kept at -40°C for 6h until the raw material disappeared. Methylamine (670g, 5.40mmol, 25% in THF) was added dropwise, then a solution of TEA (546g, 5.4mmol) in DCM (5 mL) was added dropwise. The temperature was kept at -40 °C for half an hour and then is naturally increased to an ambient temperature, and the reactants were stirred overnight. After the reaction was completed, the reactants were cooled to 0°C, a potassium carbonate solution (1g, 10 mL) was added dropwise. The reactants were extracted with DCM (10mL×3), washed with water (5mL×2), dried and concentrated, followed by high performance liquid chromatography to obtain pure Compound #5 (130mg, with a yield of 48.9%) as a pale yellow oil. ¹H-NMR(400M, CDCl₃): δ7.99(d,*J*=8.4Hz, 1H), 7.47-7.44(m, 2H), 7.24(d,J=1.6Hz, 1H), 7.07-7.04(m, 3H), 4.94(d,J=7.9Hz, 2H), 3.11-3.02(m, 6H), 2.62(s, 3H), 2.59(s, 3H). MS: Calculated 422.1, found 423.2([M+H]⁺).

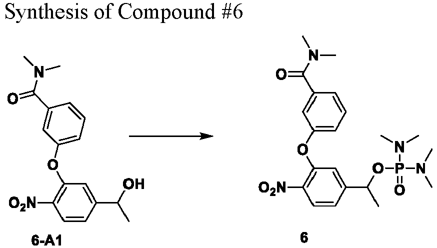

Under the protection of nitrogen, tris(dimethylamino)phosphine (179 mg, 1.1 mmol, commercially available) and tetrazole (0.64 mg, 0.009 mmol) were added to acetonitrile (3 mL), then a solution of 6-A1 (300 mg, 0.914 mmol, synthesized with reference to the synthesis method of Compound #29) in acetonitrile (2 mL) was added dropwise. The reactants were stirred at room temperature for two hours, and then TEA (277 mg, 2.74 mmol) and tert-butanol peroxide (329 mg, 3.66 mmol) were added dropwise. The reactants were stirred at room temperature for three hours, until the reaction was completed. An aqueous sodium thiosulfate solution (4 mL) was added dropwise, and the reactants were extracted with DCM (5mL×3), dried and concentrated, followed by high performance liquid chromatography to separate and obtain Compound #6 (120 mg, with a yield of 28.3%) as a pale yellow oil. ¹H-NMR(400M, CDCl₃):δ7.97(d, *J*=8.4Hz, 1H), 7.42-7.38(m, 1H), 7.23-7.20(m, 2H), 7.10-7.05(m, 3H), 5.41-5.38(m, 1H), 3.08(s, 3H), 2.98(s, 3H), 2.64(d, *J*=10.0Hz, 6H), 2.43(d, *J*=10.0Hz, 6H), 1.52(d, *J*=6.5Hz, 3H). MS: Calculated 464.2, found 465.2([M+H]⁺).

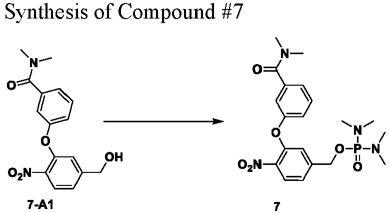

Under the protection of nitrogen, tris(dimethylamino)phosphine (124mg, 0.76mmol, commercially available) and tetrazole (0.4mg, 0.0057mmol) were added to acetonitrile (3 mL), then a solution of 7-A1 (200mg, 0.63mmol, synthesized with reference to the synthesis method of Compound #29) in acetonitrile (2 mL) was added dropwise. The reactants were stirred at room temperature for two hours. TEA (192mg, 1.90mmol) and tert-butanol peroxide (228mg, 2.53mmol) were added dropwise. The reactants were stirred at room temperature for three hours, until the reaction was completed. An aqueous sodium thiosulfate solution (4 mL) was added dropwise, and the reactants were extracted with DCM (5mL×3), dried and concentrated, followed by high performance liquid chromatography to separate and obtain Compound #7 (90 mg, with a yield of 31.6%) as a pale yellow oil. ¹H-NMR(400M, CDCl₃): 88.00(d, J=8.4Hz, 1H), 7.46-7.42(m, 1H), 7.28-7.23(m, 2H), 7.12-7.08(m, 3H), 4.97-4.95(m, 2H), 3.11(s, 3H), 3.00(s, 3H), 2.60(d, *J*=10.0Hz, 12H). MS: Calculated 450.2, found 451.2([M+H]⁺).

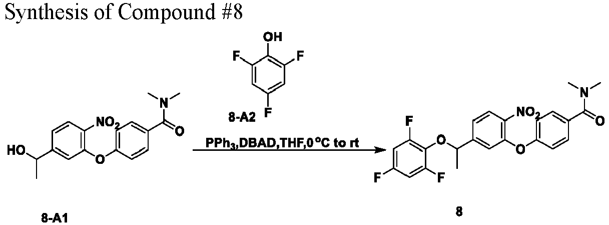

Under the protection of nitrogen, 8-A1 (300 mg, 0.91 mmol, synthesized with reference to the synthesis method of Compound #29) and 8-A2 (202 mg, 1.36 mmol) were dissolved in anhydrous THF (5 mL). The reactants were cooled to 0°C. Triphenylphosphonium (600 mg, 2.30 mmol) was added, then a solution of di-tert-butyl azodicarboxylate (530 mg, 2.30 mmol) in THF (3 mL) was slowly added dropwise. The reactants were kept at 0 °C for half an hour and then the reactants were stirred at room temperature for 2.5 hours. After the reaction was completed, water (5 mL) was added dropwise at 0 °C, and the reactants were extracted with DCM (5mL×3), washed with water (2mL×), dried and concentrated, followed by high performance liquid chromatography to obtain pure Compound #8 (230 mg, with a yield of 54.9%) as a pale yellow solid. ¹H-NMR(400M, DMSO-*d6*): δ8.13(d, *J*= 8.4Hz, 1H), 7.68(s, 2H), 7.56(dd, *J*=8.4, 2.0Hz, 1H), 7.47(dd, *J*=6.8, 2.0Hz, 2H), 7.33(d, *J*=1.6Hz, 1H), 7.05-7.03(m, 2H), 5.52-5.50(m, 1H), 2.95(s, 6H), 1.57(d, *J*=6.5Hz, 3H).

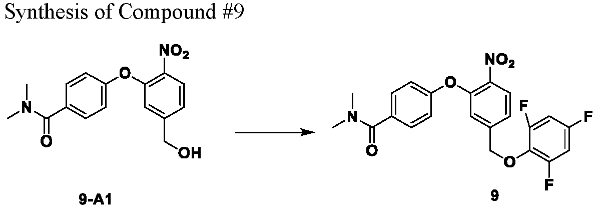

Under the protection of nitrogen, 9-A1 (300mg, 0.95 mmol, synthesized with reference to the synthesis method of Compound #29) and 2, 4, 6-trifluorophenol (210 mg, 1.40 mmol) were dissolved in dry THF (5 mL). Triphenylphosphine (622 mg, 2.40 mmol) was added, and the temperature was reduced to 0 °C. A solution of di-tert-butyl azodicarboxylate (550 mg, 2.40 mmol) in THF (4 mL) was added dropwise to react for 2.5 h, and after the reaction was completed, water (5 mL) was added. The reactants were extracted with DCM (3×15mL), washed with saline water and dried to remove the solvent, followed by high performance liquid chromatography to obtain Compound #9 (210 mg, with ayield of 49.5%) as an off-white solid. ¹H-NMR(400M, CDCl₃): δ8.02(d, J=8.4Hz, 1H), 7.46(d, *J*=8.8Hz, 2H), 7.40(d, J=8.4Hz, 1H), 7.32(s, 2H), 7.24(s, 1H), 7.07(d, J=8.8Hz, 2H), 5.01(s, 2H), 3.11(s, 3H), 3.02(s, 3H).

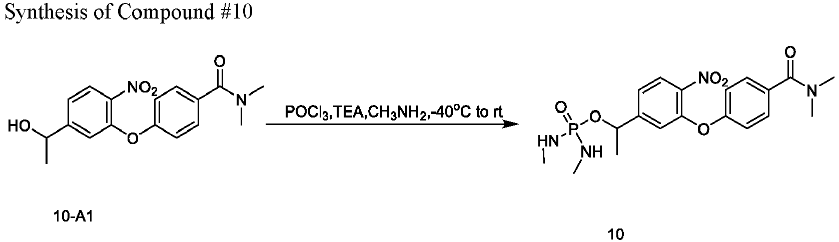

Under the protection of nitrogen, phosphorus oxychloride (348mg, 2.30mmol) was added dropwise to anhydrous DCM (5 mL). The reactants were cooled to -40°C, a solution of 10-A1 (300mg, 0.91mmol, synthesized with reference to the synthesis method of Compound #29) in DCM (3 mL) was added dropwise, then a solution of TEA (240mg, 2.40mmol) in DCM (2 mL) was added dropwise, with the temperature being kept at -40°C for 6h until the raw material disappeared. Methylamine (960mg, 7.70mmol, 25% in THF) was added dropwise, then a solution of TEA (782mg, 7.70mmol) in DCM (5 mL) was added dropwise. The temperature was kept at -40 °C for half an hour and then is naturally increased to an ambient temperature, and the reactants were stirred overnight. The reactants were cooled to 0 °C, and a potassium carbonate solution (1g, 10 mL) was added dropwise. The reactants were extracted with DCM (10mL×3), washed with water (5mL×2), dried and concentrated, followed by high performance liquid chromatography to obtain pure Compound #10 (60mg, with ayield of 15.2%) as apale yellow viscous oil. ¹H-NMR(400MHz, CDCl₃): δ7.97(d, J=8.4Hz, 1H), 7.46(d, J=8.5Hz, 2H), 7.24-7.22(m, 1H), 7.09(s, 1H), 7.05(d, J=8.5Hz, 2H), 5.48-5.44(m, 1H), 3.07(s, 6H), 2.61(d, J=12.1Hz, 3H), 2.42(d, *J*=12.1Hz, 3H), 1.54(d, *J*=6.4Hz, 3H). MS: Calculated 436.2, found 437.2([M+H]⁺).

### Synthesis of Compound #11

Under the protection of nitrogen, phosphorus oxychloride (242mg, 1.58mmol) was added dropwise to anhydrous DCM (5 mL).The reactants were cooled to -40°C, a solution of 11-A1 (200mg, 0.63mmol, synthesized with reference to the synthesis method of Compound #29) in DCM (3 mL) was added dropwise, then a solution of TEA (166mg, 1.64mmol) in DCM (2 mL) was added dropwise, with the temperature being kept at -40°C for 6h until the raw material disappeared. Methylamine (670mg, 5.40mmol, 25% in THF) was added dropwise, then a solution of TEA (546mg, 5.4mmol) in DCM (5 mL) was added dropwise. The temperature was kept at -40 °C for half an hour and then is naturally increased to an ambient temperature, and the reactants were stirred overnight. The reactants were cooled to 0 °C, and a potassium carbonate solution (1g, 10 mL) was added dropwise. The reactants were extracted with DCM (10mL×3), washed with water (5mL×2), dried and concentrated, followed by high performance liquid chromatography to obtain pure Compound #11 (95mg, with a yield of 35.7%) as a pale yellow viscous oil. ¹H-NMR(400, MeOD): δ8.04(*d*, *J*=8.4Hz, 1H), 7.49(*d,* J=8.7Hz, 2H), 7.40(d, J=8.5Hz, 1H), 7.27(s, 1H), 7.09(d, J=8.6Hz, 2H), 4.99(d, J=7.9Hz, 2H), 3.12(s, 3H), 3.07(s, 3H), 2.50(d, J=12.4Hz, 6H). MS: Calculated 422.1, found 423.2([M+H]⁺).

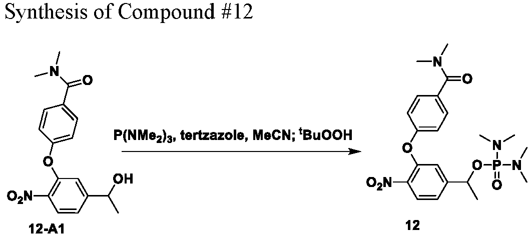

Under the protection of nitrogen, tris(dimethylamino)phosphine (179mg, 1.1mmol, commercially available) and tetrazole (0.64mg, 0.009mmol) were added to acetonitrile (3 mL), then a solution of 12-A1 (300mg, 0.91mmol, synthesized with reference to the synthesis method of Compound #29) in acetonitrile (2 mL) was added dropwise. The reactants were stirred at room temperature for two hours, and then TEA (277mg, 2.74mmol) and tert-butanol peroxide (329mg, 3.66mmol) were added dropwise. The reactants were stirred at room temperature for three hours, until the reaction was completed. An aqueous sodium thiosulfate solution (4 mL) was added dropwise, and the reactants were extracted with DCM (5mL×3), dried and concentrated, followed by high performance liquid chromatography to separate and obtain Compound #12 (76.5 mg, with a yield of 18.2%) as a pale yellow oil. ¹H-NMR(400M, CDCl₃): δ7.98(d, *J*=8.4Hz, 1H), 7.45(d, *J*=8.6Hz, 2H), 7.24(d, *J*=1.5Hz, 1H), 7.09(d, *J*=1.4Hz, 1H), 7.04(d, J=8.6Hz, 2H), 5.43-5.39(m, 1H), 3.07(s, 6H), 2.65(d, J=10.0Hz, 6H), 2.44(d, *J*=10.0Hz, 6H), 1.52(d, J=6.5Hz, 3H). MS: Calculated 464.2, found 465.2([M+H]⁺).

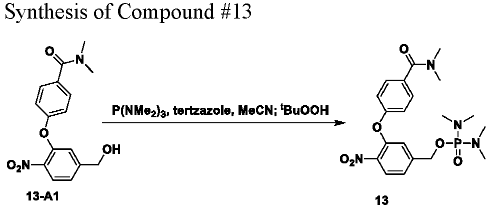

Under the protection of nitrogen, tris(dimethylamino)phosphine (124mg, 0.76mmol, commercially available) and tetrazole (0.4mg, 0.0057mmol) were added to acetonitrile (3 mL), then a solution of 13-A1 (200mg, 0.63mmol, synthesized with reference to the synthesis method of Compound #29) in acetonitrile (2 mL) was added dropwise. The reactants were stirred at room temperature for two hours, and then TEA (192mg, 1.90mmol) and tert-butanol peroxide (228mg, 2.53mmol) were added dropwise. The reactants were stirred at room temperature for two hours, until the reaction was completed. An aqueous sodium thiosulfate solution (4 mL) was added dropwise, and the reactants were extracted with DCM (5mL×3), dried and concentrated, followed by high performance liquid chromatography to separate and obtain Compound #13 (31 mg, with a yield of 9.0%) as a pale yellow oil. ¹H-NMR(400M, CDCl₃): δ7.99(d, *J*=8.4Hz, 1H), 7.45(d, *J*=8.8Hz, 2H), 7.24(m, 1H), 7.06-7.04(m, 3H), 4.93(d, *J*=7.6Hz, 2H), 3.11(s, 3H), 3.02(s, 3H), 2.62(s, 6H), 2.59(s, 6H). MS: Calculated 450.2, found 451.1([M+H]⁺).

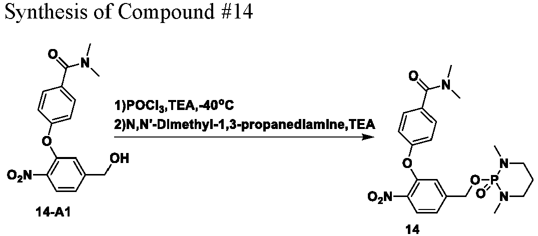

Under the protection of nitrogen, phosphorus oxychloride (246mg, 0.949mmol) was added dropwise to anhydrous DCM (10 mL). The reactants were cooled to -40°C, a solution of 14-A1 (200mg, 0.633mmol, synthesized with reference to the synthesis method of Compound #29) in DCM (4 mL) was added dropwise, then TEA (96mg, 0.949mmol) was added dropwise, and the temperature was kept at -40°C to -35 °C for 2h. As shown by the follow-up detection of HPLC, 14-A1 was exhausted and converted into an intermediate. At -40°C, a solution of N, N'- dimethyl-1, 3-propanediamine (130mg, 1.27mmol, commercially available) in DCM (2mL) was added dropwise, and then a solution of TEA (130mg, 1.266mmol) in DCM (2ml) was added dropwise, with the temperature being kept at -40 °C for one hour until the conversion of the intermediate was completed. The temperature was naturally raised to 0°C, and a saturated aqueous solution of ammonium chloride (5mL) was added dropwise. The reactants were extracted with DCM (10mL×3), washed with purified water (3ml×3), dried and concentrated, followed by HPLC to obtain Compound #14 (8.5mg, with a yield of 2.9%) as a pale yellow oily liquid. ¹H-NMR(400MHz, CDCl₃): δ7.98(d, *J*=8.4Hz, 1H), 7.45(d, *J*=8.4Hz, 2H), 7.23(d, *J*=8.5Hz, 1H), 7.11-7.02(m, 3H), 4.92(d, J=7.5Hz, 2H), 3.15-2.86(m, 10H), 2.65(s, 3H), 2.63(s, 3H), 2.03-1.97(m, 1H), 1.52-1.50(m, 1H). MS: Calculated 462.2, found 463.1([M+H]⁺).

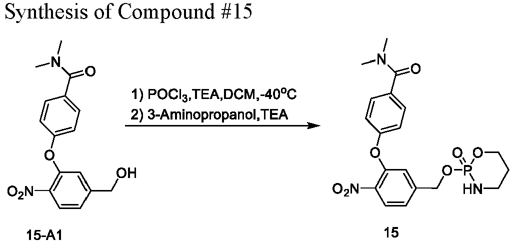

Under the protection of nitrogen, phosphorus oxychloride (53mg, 0.348mmol) was added dropwise to anhydrous DCM (10 mL). The reactants were cooled to -40°C, a solution of 15-A1 (100mg, 0.316mmol, synthesized with reference to the synthesis method of Compound #29) in DCM (2 mL) was added dropwise, then TEA (35mg, 0.348mmol) was added dropwise, and the temperature was kept at -40°C to -35 °C for 2h. As monitored by HPLC and LC-MS, 15-A1 was exhausted and converted into an intermediate. At -40°C, a solution of 3-amino-1-propanol (26mg, 0.348mmol) in DCM (2mL) was added dropwise, and then a solution of TEA (96mg, 0.948mmol) in DCM (2ml) was added dropwise, with the temperature being kept at -40°C for one hour until the conversion of the intermediate was completed. The temperature was naturally raised to 0°C, and a saturated aqueous solution of ammonium chloride (5mL) was added dropwise. The reactants were extracted with DCM (10mL×3), washed with purified water (3ml×3), dried and concentrated, followed by HPLC to obtain Compound #15 (44.5mg, with a yield of 32.3%) as a white solid. ¹H-NMR(400M, CDCl₃): δ7.99(d, J=8.4Hz, 1H), 7.46(dd, *J*=9.0, 2.2Hz, 2H), 7.27-7.25(m, 1H), 7.08(s, 2H), 7.06(d, J=2.5Hz, 1H), 5.03-5.00(m, 2H), 4.45-4.30(m, 1H), 4.23-4.16(m, 1H), 3.30-3.24(m, 1H), 3.12-3.07(m, 7H), 2.07-1.98(m, 2H), 1.67-1.62(m, 1H). MS: Calculated 435.1, found 436.1([M+H]⁺).

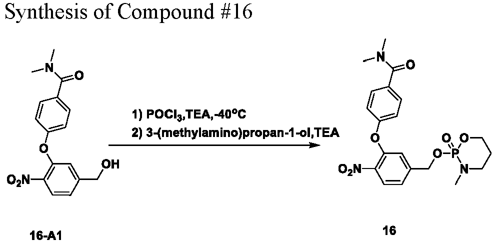

Under the protection of nitrogen, phosphorus oxychloride (54mg, 0.35mmol) was added dropwise to anhydrous DCM (10 mL). The reactants were cooled to -40°C, a solution of 16-A1 (100mg, 0.32mmol, synthesized with reference to the synthesis method of Compound #29) in DCM (2 mL) was added dropwise, then TEA (36mg, 0.35mmol) was added dropwise, and the temperature was kept at -40°C for 3h. At -40°C, a solution of 3-(methylamino)-1-propanol (31 mg, 0.35 mmol, commercially available) in DCM (2mL) was added dropwise, and then a solution of TEA (107mg, 1.05mmol) in DCM (2ml) was added dropwise, with the temperature being kept at -40 °C for 3h. The temperature was naturally raised to room temperature, and the reactants were allowed to react overnight. At 0°C, a saturated aqueous solution of ammonium chloride (5mL) was added dropwise. The reactants were extracted with DCM (8mL×3), washed with purified water (3ml×3), dried and concentrated, followed by HPLC to obtain Compound #16 (33mg, with a yield of 22.9%) as a semisolid. ¹H-NMR(400M, CDCl₃): δ7.99(d,*J*=8.4Hz, 1H), 7.46(d,*J*=8.4Hz, 2H), 7.23-7.25(m, 1H), 7.06-7.08(m, 3H), 4.94-5.12(m, 2H), 4.22-4.31(m, 1H), 4.05-4.12(m, 1H), 2.97-3.12(m, 8H), 2.65(d,*J*=10.8Hz, 3H), 2.10-2.19(m, 1H), 1.71-1.76(m, 1H). MS: Calculated 449.1, found, 450.1([M+H]⁺).

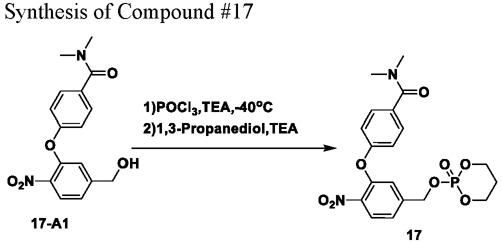

Under the protection of nitrogen, phosphorus oxychloride (53.4mg, 0.348mmol) was added dropwise to anhydrous DCM (5 mL). The reactants were cooled to -40°C, a solution of 17-A1 (100mg, 0.316mmol, synthesized with reference to the synthesis method of Compound #29) in DCM (2 mL) was added dropwise, then TEA (35.2mg, 0.348mmol) was added dropwise, and the temperature was kept at -40°C for 1.5h until the raw material is completely converted into an intermediate. At -40°C, a solution of 1, 3-propanediol (26.5mg, 0.348mmol) in DCM (2mL) was added dropwise, and then a solution of TEA (106mg, 1.043mmol) in DCM (2ml) was added dropwise, with the temperature being kept at -40°C until the reaction was completed. At 0°C, a saturated aqueous solution of ammonium chloride (3mL) was added dropwise. The reactants were extracted with DCM (5mL×3), washed with purified water (3ml×3), dried and concentrated, followed by HPLC to obtain Compound #17 (33mg, with a yield of 22.9%) as a pale yellow oily liquid. ¹H-NMR(400M, CDCl₃): δ8.00(d, J=8.4Hz, 1H), 7.47(d, *J*=8.5Hz, 2H), 7.27-7.25(m, 1H), 7.11-7.04(m, 3H), 5.10(d, *J*=8.5Hz, 2H), 4.47-4.35(m, 2H), 4.27(m, 2H), 3.07(s, 6H), 2.27(dd, J=10.9, 4.4Hz, 1H), 1.80(m, 1H), 1.34-1.18(m, 2H). MS: Calculated 436.1, found 437.1([M+H]⁺).

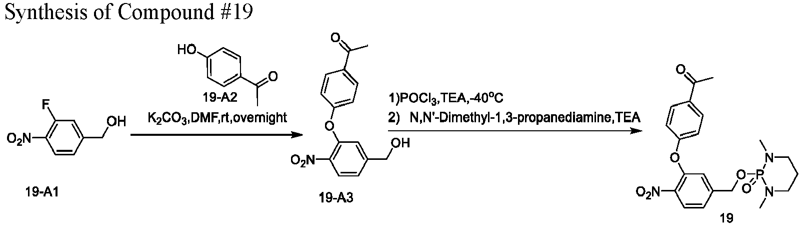

Under the protection of nitrogen, 19-A1 (2.0 g, 11.69 mmol) and 19-A2 (6.4 g, 46.76 mmol, commercially available) were dissolved in DMF (10 mL), then potassium carbonate (6.5 g, 46.76 mmol) was added, and the reactants was stirred overnight at 40 °C. After the reaction was complete, the temperature was reduced to room temperature. Water (20 ml) was added dropwise, followed by extraction with EA (20 mL×3). The substance obtained was washed with water (8ml×5) and saline water (8mL×3), dried and concentrated, followed by column separation (300-400 mesh silica gel, n-heptane: EA, 25%-35%EA) to obtain 19-A3 (1.05 g, 31.3%) as a pale yellow solid. ¹H-NMR(400MHz, CDCl₃): δ8.02(d, J=8.4Hz, 1H), 7.99-7.95(m, 2H), 7.32-7.28(m, 1H), 7.18(d, *J*=0.6Hz, 1H), 7.06-7.00(m, 2H), 4.77(s, 2H), 2.58(s, 3H). MS: Calculated 287.1, found 288.0([M+H]⁺).

Under the protection of nitrogen, phosphorus oxychloride (59mg, 0.382mmol) was added dropwise to anhydrous DCM (10 mL). The reactants were cooled to -40°C, a solution of 19-A3 (100mg, 0.347mmol) in DCM (2ml) was added dropwise, then TEA (39mg, 0.382mmol) was added dropwise, and the temperature was kept at -40°C to -35 °C for 2h. As monitored by HPLC and LC-MS, 19-A3 was exhausted and converted into an intermediate. At -40°C, a solution of N, N'- dimethyl-1, 3-propanediamine (39mg, 0.382mmol, commercially available) in DCM (2mL) was added dropwise, and then a solution of TEA (105mg, 1.041mmol) in DCM (2ml) was added dropwise, with the temperature being kept at -40 °C for one hour until the conversion of the intermediate was completed. The temperature was naturally raised to 0°C, and a saturated aqueous solution of ammonium chloride (5mL) was added dropwise. The reactants were extracted with DCM (10mL×3), washed with purified water (3ml×3), dried and concentrated, followed by HPLC to obtain Compound #19 (34.5mg, with ayield of 20.8%) as a pale yellow oily liquid. ¹H-NMR(400MHz, CDCl₃): δ8.02(d,*J*=8.4Hz, 1H), 7.97(dd, *J*=11.2, 2.4Hz, 2H), 7.30(dd, *J*=8.4, 1.0Hz, 1H), 7.15(s, 1H), 7.04(dd, *J*=11.2, 2.4Hz, 2H), 4.95(d, *J*=7.6Hz, 2H), 3.10-3.04(m, 2H), 3.01-2.88(m, 2H), 2.65(s, 3H), 2.63(s, 3H), 2.58(s, 3H), 2.04-2.01(m, 1H), 1.51-1.47(m, 1H). MS: Calculated 433.1, found 434.1([M+H]⁺).

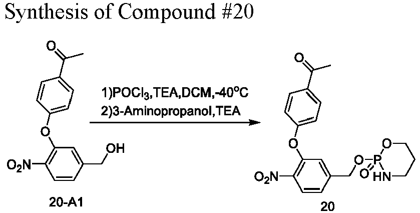

Under the protection of nitrogen, phosphorus oxychloride (59mg, 0.382mmol) was added dropwise to anhydrous DCM (10 mL). The reactants were cooled to -40°C, a solution of 20-A1(100mg, 0.347mmol, i.e., 19-A3) in DCM (2ml) was added dropwise, then TEA (39mg, 0.382mmol) was added dropwise, and the temperature was kept at -40°C to -35 °C for 2h. As monitored by HPLC and LC-MS, 20-A1 was exhausted and converted into an intermediate. At -40°C, a solution of 3-amino-1-propanol (29mg, 0.382mmol, commercially available) in DCM (2mL) was added dropwise, and then a solution of TEA (105mg, 1.041mmol) in DCM (2ml) was added dropwise, with the temperature being kept at -40 °C for one hour until the conversion of the intermediate was completed. The temperature was naturally raised to 0°C, and a saturated aqueous solution of ammonium chloride (5mL) was added dropwise. The reactants were extracted with DCM (10mL×3), washed with purified water (3ml×3), dried and concentrated, followed by HPLC to obtain the product (13.3mg, with a yield of 9.4%) as a pale yellow oily liquid. ¹H-NMR(400M, CDCl₃): δ8.03(d, *J*=8.4Hz, 1H), 7.98(d, *J*=8.8Hz, 2H), 7.35(dd, *J*=8.4, 1.1Hz, 1H), 7.18(s, 1H), 7.04(d, *J*=8.8Hz, 2H), 5.05(d, *J*=7.6Hz, 2H), 4.33-4.41(m, 1H), 4.19-4.26(m, 1H), 3.14-3.34(m, 2H), 2.58(s, 3H), 1.97-2.09(m, 1H), 1.61-1.65(m, 1H). MS: Calculated 406.1, found 407.1([M+H]⁺).

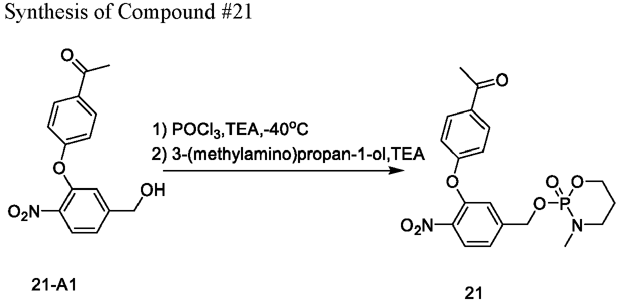

Under the protection of nitrogen, phosphorus oxychloride (59mg, 0.382mmol) was added dropwise to anhydrous DCM (10 mL). The reactants were cooled to -40°C, a solution of 21-A1(100mg, 0.347mmol, i.e., 19-A3) in DCM (2ml) was added dropwise, then TEA (39mg, 0.382mmol) was added dropwise, and the temperature was kept at -40°C to -35 °C for 2h. At -40°C, a solution of 3-(methylamino)-1-propanol (34mg, 0.382mmol) in DCM (2mL) was added dropwise, and then a solution of TEA (105mg, 1.041mmol) in DCM (2ml) was added dropwise, with the temperature being kept at -40°C for 0.5h. The temperature was naturally raised to room temperature, and the reactants were allowed to react overnight. At 0°C, a saturated aqueous solution of ammonium chloride (5mL) was added dropwise. The reactants were extracted with DCM (8mL×3), washed with purified water (3ml×3), dried and concentrated, followed by HPLC to obtain Compound #21 (41mg, with ayield of 27.2%) as a semisolid. ¹H-NMR(400M, CDCl₃): δ8.03(d,*J*=8.4Hz, 1H), 7.98(d,*J*=8.8Hz, 2H), 7.30(dd, *J*=8.4, 1.6Hz, 1H), 7.16(d, *J*=1.6Hz, 1H), 7.05(d, *J*=8.8Hz, 2H), 4.97-5.12(m, 2H), 4.23-4.33(m, 1H), 4.09-4.16(m, 1H), 2.97-3.16(m, 2H), 2.65(d, *J*=10.8Hz, 3H), 2.59(s, 3H), 2.08-2.24(m, 1H), 1.71-1.77(m, 1H). MS: Calculated 420.1, found 421.1([M+H]⁺).

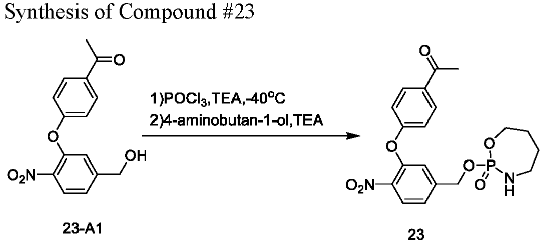

Under the protection of nitrogen, phosphorus oxychloride (59mg, 0.382mmol) was added dropwise to anhydrous DCM (10 mL). The reactants were cooled to -40°C, a solution of 23-A1(100mg, 0.347mmol, i.e., 19-A3) in DCM (2ml) was added dropwise, and then TEA (39.0mg, 0.382mmol) was added dropwise. The temperature was kept at -40°C for 1.5h, and the raw materials were completely converted into an intermediate. At -40°C, a solution of 4- amino-1-butanol (34.0mg, 0.382mmol, commercially available) in DCM (2mL) was added dropwise, and then a solution of TEA (116mg, 1. 145mmol) in DCM (2ml) was added dropwise, with the temperature being kept at -40°C. The reaction was completely after 5h. At 0°C, a saturated aqueous solution of ammonium chloride (3mL) was added dropwise. The reactants were extracted with DCM (5mL×3), washed with purified water (3ml×3), dried and concentrated to obtain Compound #23 (15.7mg, 10.7%) as a pale yellow oily liquid. ¹H-NMR(400MHz, CDCl₃): δ8.03(d, *J*=8.4Hz, 1H), 7.99(t, *J*=5.7Hz, 2H), 7.33(d, *J*=8.5Hz, 1H), 7.18(s, 1H), 7.04(d, *J*=8.7Hz, 2H), 5.07(m, 2H), 4.33-4.23(m, 1H), 4.13(m, 1H), 3.10-2.99(m, 1H), 2.90-2.83(m, 1H), 2.59(s, 3H), 1.91-1.75(m, 4H). MS: Calculated 420.1, found 421.1([M+H]⁺).

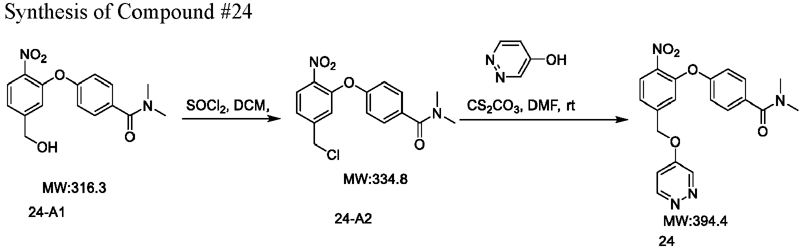

24-A1 (100 mg, 0.32 mmol) was dissolved in DCM (3 ml). The temperature was reduced to 0 °C, thionyl chloride (57.12 mg, 4.8 mmol, 1.5 eq) was added dropwise with stirring at room temperature. The reaction was completed after 1.5 h. The temperature was reduced to 0-5 °C, a saturated solution of sodium bicarbonate was added dropwise, and the pH was adjusted to be weakly alkaline (pH = 7-8). The reactants were extracted with DCM (10mL×2), dried and concentrated to obtain 24-A2 (130 mg) as a yellow oil, which was used directly into the next step.

24-A2 (130 mg, 0.388 mmol) was dissolved in DMF (3 ml). 3-hydroxypyridazine (74.57 mg, 0.776 mmol, 2 eq) was added, and cesium carbonate (316 mg, 0.96 mmol, 2.5 eq) was added with stirring at room temperature. The reaction was completed after 40 min. EA (a volume of 20 times) was added. The reactants were washed with saturated sodium carbonate (5ml×3), then with saline water, and dried and concentrated to obtain the product (26 mg, 17.0%) as ayellow oil. ¹H-NMR(400MHz, CDCl₃): δ8.05(s, 1H), 8.00(d,*J*=8.2Hz, 1H), 7.94(s, 1H), 7.49(d, *J*=7.8Hz, 2H), 7.13(d, *J*=8.2Hz, 1H), 7.09(d, *J*=7.8Hz, 2H), 6.94(s, 1H), 6.72(s, 1H), 5.18(s, 2H), 3.15(s, 3H), 3.05(s, 3H). MS: Calculated 394.1, found 394.9([M+H]⁺).

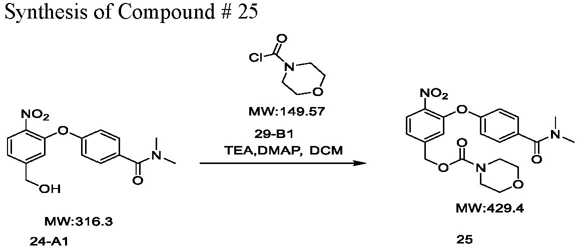

24-A1 (50 mg, 0.16 mmol) was dissolved in DCM (3 ml). 29-B1 (141.8 mg, 0.96 mmol, 6 eq) was added, and the temperature was reduced to 0 °C. TEA (95.9 mmol, 0.96 mmol, 6 eq) was added dropwise, DMAP (4.85mg, 0.04mmol, 0.25eq) was added, and the temperature was raised to 40°C with stirring overnight. After the reaction was completed, the solvent was removed, and HPLC was performed to obtain a product of 14.1 mg as a white solid, with a yield of 20.5%. ¹H-NMR(400M, CDCl₃): *δ*7.99(d, J=8.4Hz, 1H), 7.46(d, J=8.4Hz, 2H), 7.21-7.23(m, 1H), 7.06(d, J=8.4Hz, 2H), 7.01(m, 1H), 5.11(s, 2H), 3.46-3.65(m, 8H), 3.11(s, 3H), 3.02(s, 3H). MS: Calculated 429.2, found 429.9([M+H]⁺).

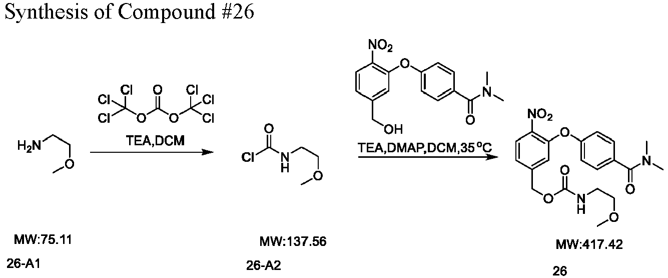

Triphosgene (3 g, 10.1 mmol) was dissolved in DCM (300 ml) at 0 °C, and then 26-A1 (1.52 g, 20.2 mmol, 2 eq) was dissolved in DCM (60 ml) followed by dropwise addition into the system over 20 min. After 4h of reaction, the system was spin-dried and slurried with MTBE (20 ml). After suction filtration, the mother liquid was spin-dried to obtain 1.2 g of 26-A2 crude product as a light brown liquid, which was directly used in the next step reaction.

26-A2 (300 mg, 0.949 mmol) and 26-A3 (652.5 mg, 4.744 mmol, 5 eq, 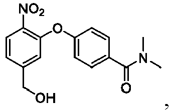 synthesized with reference to the synthesis method of Compound # 29) were dissolved in DCM (12 ml). After the temperature was reduced to 0°C, TEA (480 mg, 4.744 mmol, 5 eq) and DMAP (29.1 mg, 0.237 mmol, 0.25 eq) were added to the system. The system was warmed to 35 °C and the reaction was completed after approximately 12h. As monitored by HPLC and LCMS, after the reaction was completed, the temperature was reduced to room temperature. Saturated NaHCO₃ (15ml×2) was added for washing. The organic phase was washed with water (10 ml×2), and the aqueous phase was extracted with DCM (20 ml×1). The organic phase was spin-dried, followed by HPLC to obtain Compound #26 (150 mg, with a yield of 37.8%) as a yellow oily liquid. ¹H-NMR(400M, CDCl₃): *δ*7.94(d, J=8.4Hz, 1H), 7.43(t, J=8.4Hz, 2H), 7.19(d, J=8.4Hz, 1H), 7.02(t, J=2.4Hz, 3H), 5.24(s, 1H), 5.06(s, 2H), 3.42(t, J=4.8Hz, 2H), 3.34(d, J=2.8Hz, 2H), 3.32(d, J=5.6Hz, 3H), 3.08(s, 3H), 3.00(s, 3H). MS: Calculated 417.2, found 418.0([M+1]⁺).

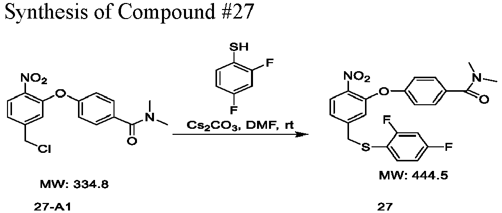

Under the protection of nitrogen, 27-A1 (120 mg, 0.358 mmol, synthesized with reference to the synthesis method of Compound #29) was dissolved in DMF (5 mL). 2, 4 difluorothiophenol (104 mg, 0.716 mmol, 2 eq) was added, and Cs₂CO₃ (291.6 mg, 0.895 mmol, 2.5 eq) was added. The reactants were stirred at ambient temperature, and the reaction was completed after 1h. EA (50 mL) was added. The reactants were washed with aqueous saturated sodium carbonate (20mlx3) and saline water (10ml×2), and dried and concentrated to obtain the product (41 mg, with a yield of 25.7%) as a yellow oily liquid. ¹H-NMR(400M, CDCl₃): *δ*7.89(d, J=8.4Hz, 1H), 7.44(d, J=8.4Hz, 2H), 7.20-7.24(m, 1H), 7.06-7.08(m, 1H), 6.92(d, J=8.4Hz, 2H), 6.79-6.82(m, 3H), 3.93(s, 2H), 3.12(s, 3H), 3.03(s, 3H). MS: Calculated 444.1, found 444.9([M+1]⁺).

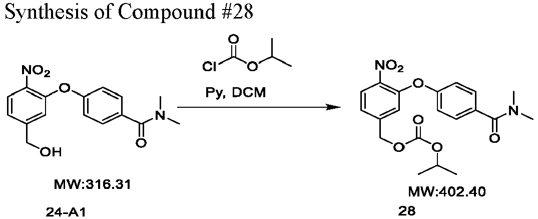

24-A1 (100 mg, 0.32 mmol, synthesized with reference to the synthesis method of Compound #29) and pyridine (55.7 mg, 0.70 mmol, 2.2 eq) were dissolved in DCM (3 ml), then cooled to 0°C. Isopropyl chloroformate (129.6 mg, 1.2 mmol, 3.6 eq, commercially available) was added to the system. The reactants were reacted at 20°C for 18 h, and cooled to 0°C-5°C. IN of hydrochloric acid (3 ml) was added dropwise, followed by extraction with DCM (10 mLx2). The organic phase was washed with hydrochloric acid (IN, 10 mLx5), water (5 mLx3) and saline water (5 mLx2), and dried over sodium sulfate, concentrated and neutralized to obtain a product (27 mg, 21.2%) as an off-white solid. ¹H-NMR(400M, CDCl₃): *δ*7.98(d, J=8.4Hz, 1H), 7.46(d, J=8.4Hz, 2H), 7.26(s, 1H), 7.04-7.07(m, 3H), 5.11(s, 2H), 4.87(q, J=6.4Hz, 1H), 3.11(s, 3H), 3.03(s, 3H), 1.29(d, J=6.4Hz, 6H). MS: Calculated 402.1, found 403.0([M+1]⁺).

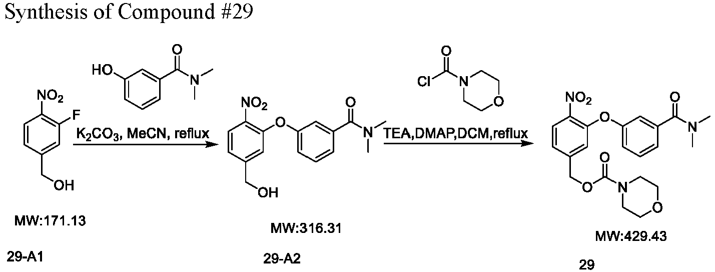

29-A1 (1 g, 5.84 mmol) and 3-hydroxy-N, N-dimethylbenzamide (2.8 g, 17.53 mmol, 3 eq) were dissolved in MeCN (30 ml), then K₂CO₃ (2.8 g, 20.45 mmol, 3.5 eq) was added to the system, and the system was warmed to 65 °C. The reaction was completed after 3 h, and then the system was cooled to 20 °C. H₂O (15 ml) was added, followed by extraction with EA (30 mL×3). The organic phase was washed with IN of NaOH solution (50 ml×3), spin-dried, and the sample was mixed, followed by 200-300 silica gel (EA: PE, 1: 3) to obtain 29-A2 (m=950 mg, with a yield of 51.4%). ¹H-NMR(400M, CDCl₃): *δ*7.96(d, J=8.4Hz, 1H), 7.39-7.43(m, 1H), 7.19-7.22(m, 2H), 7.09-7.11(m, 1H), 7.04-7.07(m, 2H), 4.69(s, 2H), 3.00(brs, 6H), 1.97(s, 1H). MS: Calculated 316.1, found 317.1[(M+1)⁺].

29-A2 (50 mg, 0.158 mmol) and piperidine-1-formyl chloride (141.9 mg, 0.948 mmol, 6 eq) were dissolved in DCM (2 ml), then TEA (32.0 mg, 0.316 mmol, 2 eq) and DMAP (4.9 mg, 0.04 mmol, 0.25 eq) were added to the system, at which moment the system is pale yellow. The system was warmed to 40 °C and the reaction was completed after 12h. The solvent was removed, and HPLC was carried out to obtain 16.5 mg of the pure product, with a yield of 24.3%. ¹H-NMR(400M, CDCl₃): *δ*7.97(d, J=8.4Hz, 1H), 7.40-7.44(m, 1H), 7.18-7.23(m, 2H), 7.08-7.10(m, 1H), 7.01-7.06(m, 2H), 5.11(s, 2H), 3.64(s, 4H), 3.46(s, 4H), 3.11(s, 3H), 2.98(s, 3H). MS: Calculated 429.2, found 330.0([M+H]⁺).

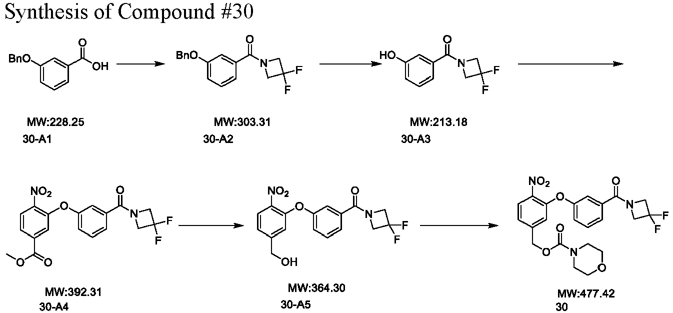

Under the protection of nitrogen, 30-A1 (1.76 g, 7.72 mmol) and 3, 3-difluoroazetidine hydrochloride (1.0 g, 7.72 mmol, leq) were dissolved in DCM (20 ml). A solution of T₃P in EA (10 g, 15.4 mmol, 2 eq, 50%) was added and the system was cooled to 5 °C. DIEA was added dropwise, followed by stirring at room temperature. As monitored by HPLC, after the reaction has completed for 2h, water (5 ml) was added to the system, followed by extraction with DCM (15 ml×3). The organic phase was washed with water (10 ml×2), spin-dried to obtain a crude product (m = 2.5 g, with a yield of 100%) as a pale yellow solid. ¹H-NMR(400M, DMSO-*d6*): *δ*7.45-7.47(m, 2H), 7.38-7.42(m, 3H), 7.31-7.35(m, 1H), 7.24-7.26(m, 2H), 7.18-7.21(m, 1H), 5.16(s, 2H), 4.47-4.70(m, 4H). Calculated 303.1, found 304.1([M+H]⁺).

Under the protection of nitrogen, 30-A2 (2.5 g, 8.242 mmol) was dissolved in EtOH (37 ml), and then Pd/C (630 mg, 25% A2) was added to the system. The system was flushed with N₂ for three times to fill the system with N₂, then flushed with H₂ for three times to fill the system with H₂, followed by stirring at ambient temperature. As monitored by HPLC, the reaction was completed after 8h. Under the protection of nitrogen, H₂ was drawn off, then Pd/C was filtered off with suction. The system was washed with DCM (30 ml×3) and the mother liquid was spin-dried to obtain 30-A3 crude product (1.5 g, with a yield of 85.4%) as an off-white solid. ¹H-NMR(400M, DMSO-*d6*): *δ*7.24-7.28(m, 1H), 7.05-7.09(m, 2H), 6.92-6.94(m, 1H), 4.46-4.69(m, 4H). Calculated 213.1, found 214.1([M+H]⁺).

30-A3 (1.5 g, 7.04 mmol) and methyl 3-fluoro-4-nitrobenzoate (2.8 g, 14.06 mmol, 2 eq) were dissolved in ACN (15 ml), and then K₂CO₃ (2.9 g, 21.01 mmol, 3 eq) was added to the system. The system was fluxed at 60 °C overnight. The reaction was completed the next day as monitored by HPLC, and the temperature was reduced to room temperature. Water (10 ml) was added, and the system was extracted with DCM (20 mL×4). The organic phase was washed with water (10 ml×2) and saline water, then spin-dried to obtain 30-A4 crude product (m = 1.8 g, with a yield of 65.2%) as yellow solid. ¹H-NMR(400M, CDCl₃): *δ*7.99(d, J=8.4Hz, 1H), 7.92(dd, J₁=8.4Hz, J₂=1.6Hz, 1H), 7.70(d, J=1.6Hz, 1H), 7.43-7.51(m, 2H), 7.29-7.30(m, 1H), 7.20-7.23(m, 1H), 4.50-4.56(m, 4H), 3.92(s, 3H).Calculated MS: 392.1, found 393.0([M+H]⁺).

30-A4 (500 mg, 1.27 mmol) was dissolved in THF (2 ml) and then the mixture was cooled to 0 °C. NaBH₄ (193 mg, 5.10 mmol, 4 eq) was added in batches to the system and the system was warmed to 60 °C to reflux. The reaction was completed after 15h as monitored by HPLC. The temperature was reduced to 0 °C and the reaction was quenched with water (20 ml). The system was extracted with DCM (20 ml×3), spin-dried and concentrated, and the sample was mixed, followed by 300-400 silica gel passed through Flash column chromatography to obtain the pure product 30-A5 (140 mg, with a yield of 30.2%) as an off-white solid. ¹H-NMR(400M, CDCl₃): *δ*7.96(d, J=8.4Hz, 1H), 7.34-7.38(m, 1H), 7.15-7.18(m, 2H), 7.04-7.06(m, 2H), 6.97(dd, J₁=8.4Hz, J₂=2.0Hz, 1H), 4.63-4.68(m, 4H), 3.67-3.76(m, 2H).Calculated 364.1, found 365.1([M+H]⁺).

30-A5 (140 mg, 0.384 mmol) and piperidine-1-carbonyl chloride (287.4 mg, 1.92 mmol, 5 eq) were dissolved in DCM (6 ml). After cooling to 0 °C, TEA (194.3 mg, 1.92 mmol, 5 eq) and DMAP (11.8 mg, 0.096 mmol, 0.25 eq) were added to the system and then the system was warmed to 35 °C. The reaction was completed after about 12 h. After the reaction was completed as monitored by HPLC and LCMS, the temperature was reduced to room temperature. Saturated NaHCO₃ (10 ml×2) was added for washing, the organic phase was washed with water (10 ml×2) and the aqueous phase was extracted with DCM (10 ml). The organic phase was spin-dried and then separated by HPLC under neutral conditions to obtain the pure product (22.5 mg, with a yield of 12.3%) as awhite solid. ¹H-NMR(400M, CDCl₃): *δ*8.00(d, J=8.8Hz, 1H), 7.40-7.48(m, 2H), 7.19-7.26(m, 3H), 7.02(s, 1H), 5.13(s, 2H), 4.49-4.55(m, 4H), 3.64(brs, 4H), 3.46(s, 4H). Calculated 477.1, found 478.0([M+H]⁺).

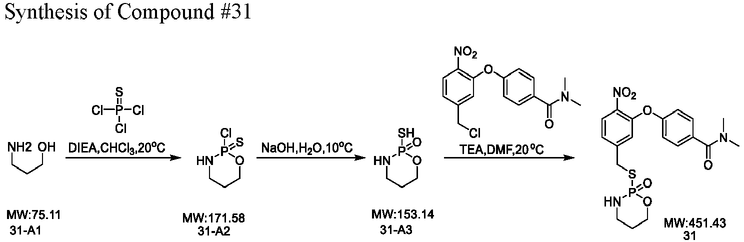

PSCl₃ (4.5 g, 26.6 mmol) was dissolved in chloroform (50 ml), then 31-A1 (2 g, 26.6 mmol) was dissolved in chloroform (40 ml) and added dropwise to the system over 1h. After stirring for 1h, DIEA (3.44 g, 26.6 mmol) was dissolved in chloroform (10 ml) and added dropwise to the system, and the system was warmed to 20 °C overnight. Water (20 ml×4) was added for extraction and the organic phase was spin-dried to obtain 31-A2 crude product (2.4 g) as an off-white solid.

31-A2 (1.8 g, 10.5 mmol) was added to H₂O (18 ml) to produce a white suspension, and then NaOH (923 mg, 23.1 mmol, 2.2 eq) was slowly added in batches to the system, after which the system gradually became clear. There was a desired product as monitored by LCMS. After reacting overnight, MeCN (20 ml) was added and mixed, and then moisture was partially removed by spin-drying. The remaining mixture was adjusted to acidic with 12 N of HCl, and a large amount of solids were precipitated. After suction filtration, the resultant was slurried with MeCN (10ml) and the moisture was removed. After suction filtration, MeCN (10ml) was added again and spin-dried to obtain 800mg of 31-A3 in total as a white solid, which was directly used in the next reaction.

Under the protection of nitrogen, 31-A4 (100 mg, 0.299 mmol, 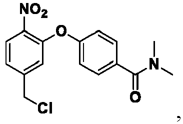 synthesized with reference to the synthesis method of Compound #29) was dissolved in DMF (2 ml), then 31-A3 (91.5 mg, 0.597 mmol, 2 eq) and TEA (90.7 mg, 0.896 mmol, 3 eq) were added to the system, after which the system was pale orange yellow. The reaction was performed overnight at 20 °C until the reaction was completed. A saturated aqueous solution of NaHCO₃ (5 ml) was added, followed by extraction with EA (5 ml×3). The organic phase was washed with water (10 ml×5) and the aqueous phase was washed with EA (10 ml×2). The organic phase was spin-dried, and subjected to HPLC in neutral conditions. After separation, the solution was lyophilized to obtain the pure product (67.4 mg, with a yield of 50.0%) as an off-white solid. ¹H-NMR(400M, CD₃OD): *δ*7.99(d, J=8.4Hz, 1H), 7.48-7.50(m, 2H), 7.40(dd, J₁=8.4Hz, J₂=1.6Hz, 1H), 7.27(d, J=1.6Hz, 1H), 7.08-7.10(m, 2H), 4.21-4.26(m, 2H), 4.03-4.07(m, 2H), 3.16-3.25(m, 2H), 3.10(s, 3H), 3.05(s, 3H)1.94-2.04(m, 1H), 1.67-1.71(m, 1H). MS: Calculated 451.1, found 452.1([M+H]⁺).

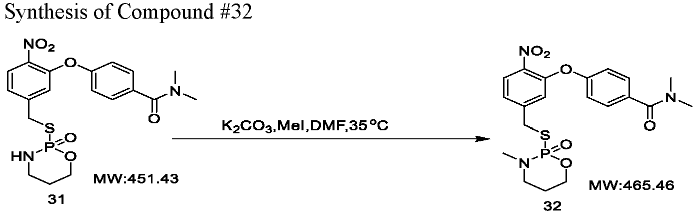

31 (20 mg, 0.044 mmol) was dissolved in DMF (1 ml), and immediately K₂CO₃ (12.2 mg, 0.088 mmol, 2 eq) was added to the reaction system. The system was stirred at room temperature for 1h. Then, MeI (22 mg, 0.132 mmol, 3 eq) was added dropwise to the system, after which there was no change after 45% conversion rate was monitored. Additional K₂CO₃ (18.3 mg, 0.132 mmol, 2 eq) and Mel (22 mg, 0.132 mmol, 3 eq) was added and the system was warmed to 35 °C. The conversion rate reached 75% after overnight. After cooling to 10 °C, a saturated aqueous NaHCO₃ solution (2 ml) was added to the system. Extraction was carried out using EA (3 ml×3), and the organic phase was washed with saline water (2 ml×3), spin-dried and then separated by HPLC under neutral conditions to obtain 6.1 mg of 32 in total as a pale yellow solid. ¹H-NMR(400M, CD₃OD): *δ*7.99(d, J=8.4Hz, 1H), 7.48-7.50(m, 2H), 7.39-7.41(m, 1H), 7.27(d, J=1.6Hz, 1H), 7.09-7.11(m, 2H), 4.06-4.10(m, 2H), 4.03-4.10(m, 2H), 3.05-3.10(m, 8H), 2.60(d, J=7.2Hz, 3H), 2.07-2.17(m, 1H), 1.68-1.80(m, 1H).

MS: Calculated 465.1, found 466.1([M+1]⁺).

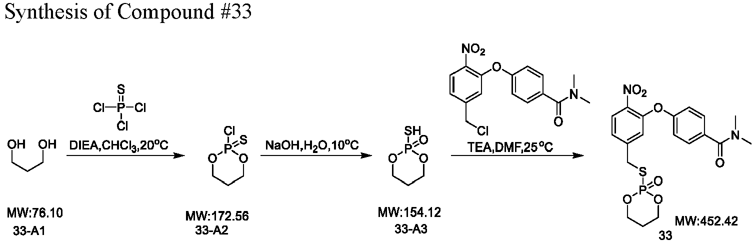

PSCl₃ (4.5 g, 26.3 mmol) was dissolved in chloroform (50 ml), then 33-A1 (2 g, 26.3 mmol) was dissolved in chloroform (40 ml) and added dropwise to the system over 1h. After stirring for 1h, DIEA (3.4 g, 26.3 mmol) was dissolved in chloroform (10 ml) and added dropwise to the system. The system was warmed to 20 °C and reacted overnight. Water was added for extraction (20 ml×4) and the organic phase was spin-dried to obtain 33-A2 crude product (3.5 g) as an off-white solid, which was directly used in the next step reaction.

33-A2 (1 g, 5.86 mmol) was added to H₂O (10 ml), then NaOH (281 mg, 7.03 mmol, 1.2 eq) was slowly added in batches to the system and reacted overnight. The desired product was monitored by LCMS. The system was adjusted to pH =3-4 with 12 N of HC1, no solid was precipitated. The moisture was removed to obtain 500 mg of 33-A3 crude product as a pale yellow liquid.

Under the protection of nitrogen, 33-A4 (100 mg, 0.299 mmol, 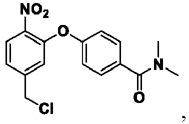 synthesized by reference to the synthesis method of Compound #29) was dissolved in DMF (2 ml), and then 33-A3 (184.3 mg, 1.196 mmol, 4 eq) and TEA (121.0 mg, 1.196 mmol, 4 eq) were added to the system. After reacting overnight at room temperature, the reaction was complete as monitored by HPLC. Saturated NaHCO₃ (10 mL) was added, and extraction was carried out with EA (5 ml×3). The organic phase was washed with water (5 ml×2) and the aqueous phase was extracted with EA (5 ml×3). The organic phase was spin-dried and subjected to HPLC under neutral conditions to obtain 29.7 mg of Compound #33 in total as a yellow solid, with a yield of 22.0%. ¹H-NMR(400M, CD₃OD): *δ*8.00(d, J=8.4Hz, 1H), 7.48-7.51(m, 2H), 7.42(dd, J=8.4Hz, 2.0Hz, 1H), 7.28(d, J=1.6Hz, 1H), 7.10-7.11(m, 2H), 4.34-4.41(m, 4H), 4.15(d, J=16.8Hz, 2H), 3.10(s, 3H), 3.05(s, 3H), 2.22-2.27(m, 1H), 1.79-1.85(m, 1H). MS: Calculated 452.1, found 453.0([M+1]⁺).

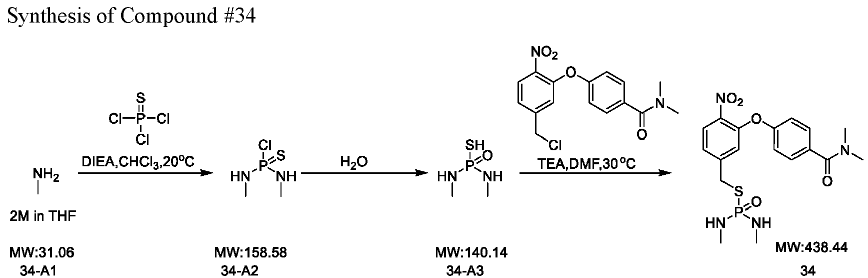

PSCl₃ (2 g, 11.8 mmol) was dissolved in chloroform (25 ml) at 0 °C, then 34-A1 (11.8 ml, 23.6 mmol, 2 eq, 2 MinTHF) was added dropwise to the system and after which the system was stirred for 1 h. Then, DIEA (3.44 g, 26.6 mmol) was dissolved in chloroform (10 ml) and then added dropwise to the system. The system was warmed to 20 °C to react overnight. The solvent was removed to obtain 34-A2 crude product. Water (20 ml) was added and stirred for 30 min, then the spin drying was carried out to obtain 5g of 34-A3 crude product as a colorless oily liquid.

Under the protection of nitrogen, 34-A4 (50 mg, 0.149 mmol, 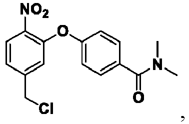 synthesized with reference to the synthesis method of Compound #29) was dissolved in DMF (1 mL), then 34-A3 (418.6 mg, 1.194 mmol, 8 eq) and TEA (120.8 mg, 1.194 mmol, 8 eq) were added to the system. The system was warmed to 30 °C and reacted overnight. After the reaction was completed as monitored by HPLC and LCMS, the temperature was reduced to room temperature. A saturated aqueous solution of NaHCO₃ (5 mL) was added, followed by extraction with EA (5 mL×3). The organic phase was washed with water (5 mL×3) and saline water, dried over sodium sulfate, spin-dried, and subjected to HPLC under neutral conditions to obtain the pure product (20.0 mg, with a yield of 30.6%) as a light yellow solid. ¹H-NMR(400M, CD₃OD): *δ*7.99(d, J=8.4Hz, 1H), 7.48-7.50(m, 2H), 7.40(dd, J=8.4Hz, 2.0Hz, 1H), 7.26(d, J=1.6Hz, 1H), 7.07-7.10(m, 2H), 3.96(d, J=12.8Hz, 2H), 3.10(s, 3H), 3.05(s, 3H), 2.51(s, 3H), 2.48(s, 3H). MS: Calculated 438.1, found 439.1([M+1]⁺).

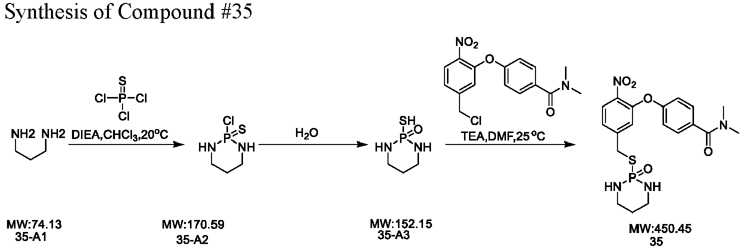

PSCl₃ (4.56 g, 26.9 mmol) was dissolved in chloroform (50 ml), then a solution of 35-A1 (2 g, 26.9 mmol) in chloroform (40 ml) was added dropwise to the system over 30 min. After stirring for 1h, a solution of DIEA (3.48 g, 26.9 mmol) in chloroform (10 ml) was added dropwise and the system was warmed to 20 °C overnight. After spinning dry, 35-A2 crude product was obtained. Water (20 ml) was added and the system was stirred for 30 min. Water was removed to obtain a white solid. MeCN (3 ml) was used for slurry and then filtration was carried out with suction to obtain 1.2 g of dry 35-A3 as a white solid, which was directly used in the next step reaction.

Under the protection of nitrogen, 35-A4 (100 mg, 0.299 mmol, 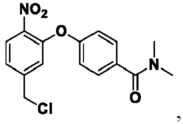 synthesized with reference to the synthesis method of Compound #29) was dissolved in DMF (2 ml), then 35-A3 (182 mg, 1.196 mmol, 4 eq) and TEA (121 mg, 1.196 mmol, 4 eq) were added to the system. The system was warmed to 25 °C and the reaction was complete after reacting overnight as monitored by HPLC. A saturated aqueous solution of NaHCO₃ (5 ml) was added and extraction was carried out with EA (5 mL×3). The organic phase was washed with water (10 ml×2) and the aqueous phase was extracted with EA (5 ml×2). The organic phase was spin-dried and then separated by HPLC under neutral conditions to obtain 23.1 mg of Compound #35 in total as a pale yellow solid, with a yield of 17.2%. ¹H-NMR(400M, CD₃OD): *δ*7.98(d, J=8.4Hz, 1H), 7.47-7.50(m, 2H), 7.40(dd, J₁=8.4Hz, J₂=1.6Hz, 1H), 7.28(d, J=1.6Hz, 1H), 7.07-7.10(m, 2H), 3.99(d, J=12.4Hz, 2H), 3.05-3.24(m, 10H), 1.69-1.82(m, 1H), 1.60-1.65(m, 1H). MS: Calculated 450.1, found 451.1([M+1]⁺).

### Compound #1

Compound #1 was synthesized in a similar manner to Compound #2/3 above.

¹H-NMR (400 MHz, CDCl3): δ 7.91 (d, J = 8.4 Hz, 1H), 7.41 (t, J = 7.9 Hz, 1H), 7.33-7.27 (m, 1H), 7.21 (d, J = 7.6 Hz, 1H), 7.15-6.98 (m, 3H), 4.55 (s, 2H), 3.54 (s, 3H), 3.36 (s, 3H), 3.09 (s, 3H), 2.97 (s, 3H).

### In vitro AKR1C3 enzyme activity inhibition experiments

Experimental apparatus:
Waters Acquity I Class UPLC Ultra Performance Liquid Chromatograph equipped with a Xevo G2-XSQ Tof HRMS Quadrupole Time-of-flight High Resolution Mass Spectrometer.

Buffers and materials:
1. PBS phosphate buffered saline solution
2. 20 mM NADPH in PBS phosphate buffered saline solution
3. 250 µg/mL AKR1C3 in PBS phosphate buffered saline solution
4.250 µM test compound in 50% MeOH/H₂O
5.250 µM progesterone in 50% MeOH/ H₂O
6.1 (µg/mL propranolol in 100% acetonitrile

### Experimental Procedures

In step 1, the reaction mixtures were prepared into Eppendorf tubes in duplicate (n=2) according to the table below and mixed gently.

| Materials | Negative controls (µL) | Samples (µL) |
|---|---|---|
| PBS | 68 | 66 |
| NADPH (20 mM) | 10 | 10 |
| AKR1C3 (250 µg/mL) | 10 | 10 |
| Test compound (250 µM) | 0 | 2 |

In step 2, the above mixtures in duplicate were pre-incubated at 37 °C for 30 minutes.

In step 3, an additional 10 µL of 20 mM NADPH in PBS phosphate buffered salt solution and 2 µL of 250 µM progesterone in 50% MeOH/H₂O were added to each Eppendorf tube and gently mixed.

In step 4, 50 µL of the mixture from the above step was immediately transferred to 100 µL of 1 (µg/mL propranolol (internal standard, IS) in 100% acetonitrile.

In step 5, the remaining sample was incubated at 37 °C for 30 minutes and 100 µL of 1 (µg/mL propranolol (internal standard, IS) in 100% acetonitrile was added.

In step 6, for all the samples, 100 µL of reagent water was added, vortex-mixed at 1100 rpm for 5 minutes, and centrifuged at 15, 000 rpm for 10 minutes at room temperature.

In step 7, all the samples were loaded on LC/MS to determine the content of reduced progesterone, i.e. 20α-dihydroprogesterone.

The test conditions for the LC-MS apparatus were as follows:

| Items | Conditions |
|---|---|
| Apparatus: | Waters Acquity I Class Liquid Chromatograph |
| Chromatographic column: | Acquity UPLC BEH C18 Chromatographic Column (50^{∗}2. 1 mm, 1.7µm) |
| Flow rate: | 0.4 mL/min |

| | |
|---|---|
| Injection volume: | 3µL |
| The composition of the mobile phase: | A: 0.1% (V/V) aqueous formic acid solution |
| | B: 0.1% (V/V) formic acid in acetonitrile |
| The temperature of the column oven: | 40 °C |
| Detector: | Quadrupole Time-of-flight Mass Spectrometer Q-TOFMS |

The elution gradient of the liquid phase

| Time (min) | A (%) | B (%) |
|---|---|---|
| 0.00 | 90.0 | 10.0 |
| 1.5 | 5.0 | 95.0 |
| 2.00 | 5.0 | 95.0 |
| 2.30 | 90.0 | 10.0 |
| 3.00 | 90.0 | 10.0 |

Parameters of the Quadrupole Time-of-flight Mass Spectrometer

| Items | Parameters |
|---|---|
| Capillary Voltage (CapilarykV) | 2.5 |
| Sampling Cone Voltage (Sampling Cone V) | 40 |
| Source Temperature (°C) | 100 |
| **Flow Rate of** Sampling Cone Gas (L/h) | 50 |
| **Flow Rate of** Desolvation Gas (L/h) | 600 |
| InterfaceType | ES, Positive |
| Analyser Mode | Sensitivity |
| Scan Range | 50-800 m/z |

In step 9, calculation of reduced progesterone (20α-dihydroprogesterone): the peak areas of the reduced progesterone i.e., 20α-dihydroprogesterone and propranolol, in each sample were determined by LC/MS. The ratio of the peak area of reduced progesterone to that of propranolol was calculated and the ratio was set to 0% when the time is 0.

AKR1C3 activity (%) = [(the amount of reduced progesterone after normalization of the sample) ₃₀ₘᵢₙ - (the amount of reduced progesterone after normalization of the sample) ₀ₘᵢₙ] / [(the amount of reduced progesterone after normalization of the negative control group) ₃₀ₘᵢₙ - (the amount of reduced progesterone after normalization of the negative control group) ₀ₘᵢₙ] ^{∗} 100.

The AKR1C3 relative activity% results for the compounds in the table below at a concentration of 5 µM/L were calculated according to the above formula.

| Serial No. | The structures of the compound | Relative Activity% | Serial No. | The structures of the compound | Relative Activity % |
|---|---|---|---|---|---|
| 1 | 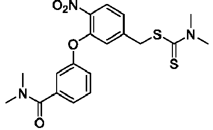 | 7% | 20 | 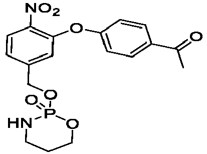 | 51.1% |
| 2 | 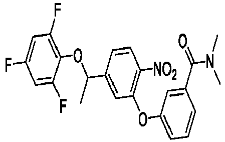 | 78% | 21 | 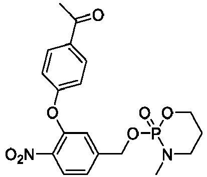 | 61.4% |
| 3 | 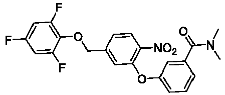 | 34% | 23 | 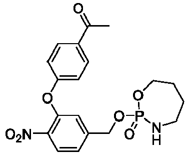 | 41.4% |
| 4 | 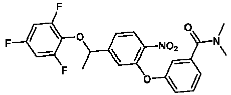 | 58% | 24 | 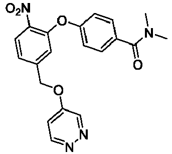 | 53.2% |
| 5 | 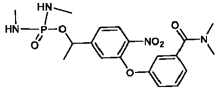 | 9% | 25 | 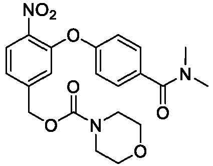 | 28.0% |
| 6 | 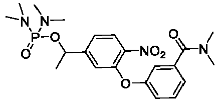 | 58% | 26 | 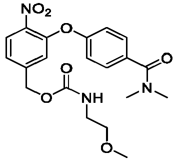 | 43.8% |
| 7 | 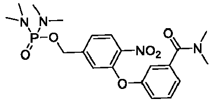 | 5% | 27 | 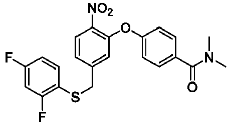 | 28.4% |
| 8 | 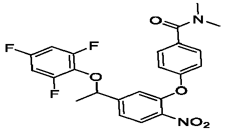 | 69% | 28 | 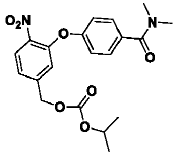 | 38.6% |
| 9 | 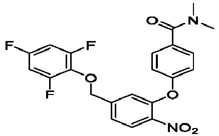 | 60% | 29 | 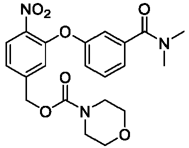 | 15.6% |
| 10 | 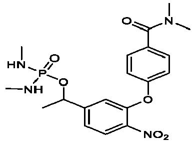 | 96% | 30 | 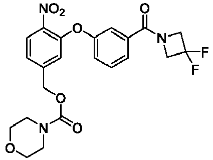 | 44.4% |
| 11 | 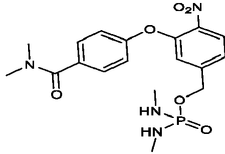 | 35% | 31 | 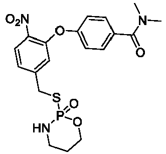 | 58.6% |
| 12 | 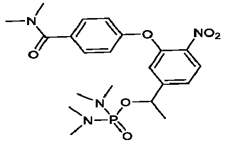 | 15% | 32 | 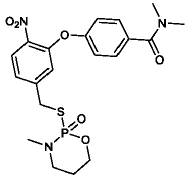 | 43.7% |
| 13 | 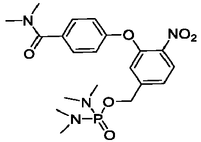 | 68.3% | 33 | 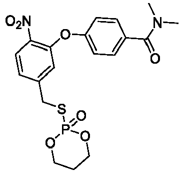 | 41% |
| 14 | 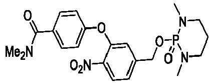 | ND | 34 | 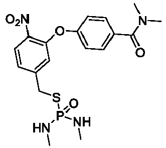 | 71.1% |
| 15 | 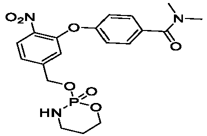 | 57.3% | 35 | 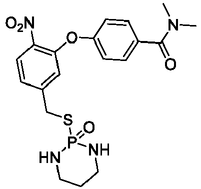 | 80.1% |
| 16 | 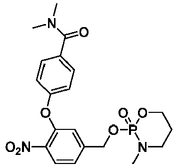 | 43.4% | 36 | 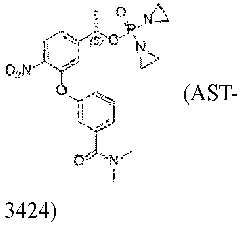 | 54.2% |
| 17 | 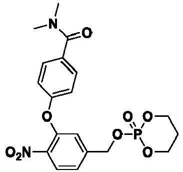 | 41.4% | 37 | Indomethacin | 92.4% |
| 19 | 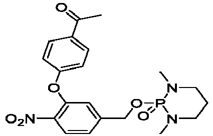 | 89.1% | | | |

Remarks:
ND, not detected.

The activity data of the above compounds were obtained from four tests: 2019/5/29, 2019/5/31, 2019/6/13, 2019/6/14, for which the corresponding control compound AST-3424 and indomethacin also have four values, and the arithmetic mean of these four values is used in the above table. The results of the tests and the time for test are specified as follows:

| Compounds and Test Concentrations | 2019/5/29 | 2019/5/31 | 2019/6/13 | 2019/6/14 | Average |
|---|---|---|---|---|---|
| AST-3424, 5µM/L | 68.1 | 73.8 | 39.9 | 35 | 54.2 |
| Indomethacin, 5 µM/L | 86.9 | 95 | 96.7 | 91 | 92.4 |

Indomethacin is a typical AKR1C3 inhibitor and is used clinically for the relief and treatment of pain including cancer pain. The compound disclosed in the present application has a higher ability to inhibit AKR1C3 enzyme at a concentration of 5 µM/L than indomethacin, showing that the compound disclosed in the present application is a more potent inhibitor of AKR1C3.

## Claims

1. A compound of the following formula and a pharmaceutically acceptable salt, or a solvate, or an isotopically substituted compound thereof: 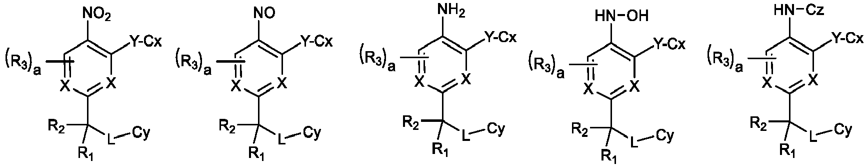
wherein
R₁ and R₂ are each independently hydrogen, deuterium, aryl or Z-substituted aryl, heteroaryl or Z-substituted heteroaryl, C₁-C₆ alkyl or Z-substituted alkyl, C₂-C₆ alkenyl or Z-substituted alkenyl, C₂-C₆ alkynyl or Z-substituted alkynyl, or C₃-C₈ cycloalkyl or Z-substituted cycloalkyl;
R₃ is hydrogen, halogen, cyano or isocyanato, hydroxyl, mercapto, amino, oximido, hydrazono, OTs, OMs, C₁-C₆ alkyl or Z-substituted alkyl, C₂-C₆ alkenyl or Z-substituted alkenyl, C₂-C₆ alkynyl or Z-substituted alkynyl, C₃-C₈ cycloalkyl or Z-substituted cycloalkyl, C₆-C₁₀ aryl or Z-substituted aryl, 4-15-membered heterocyclic radical or Z-substituted heterocyclic radical, 5-15-membered heteroaryl or Z-substituted heteroaryl, or C₁-C₆ alkoxy or Z-substituted C₁-C₆ alkoxy; or R₃ is -CONR⁶R⁷, -SO₂NR⁶R⁷, -SO₂R⁶, -OCO-R⁶, -OCOO-R⁶, -COOR⁶, -NR⁶COR⁷, -NR⁶SO₂R⁷, or -NR⁶CONR⁶R⁷; and R⁶ and R⁷ together with N to form or not to form a 4-8-membered Z-substituted heterocyclic ring, or two R₃ together with the atom on a benzene ring to which they are bonded to form a 7-15-membered fused ring or a Z-substituted fused ring;
R⁶ and R⁷ are each independently hydrogen, cyano or isocyanato, C₁-C₆ alkyl or Z-substituted alkyl, C₂-C₆ alkenyl or Z-substituted alkenyl, C₂-C₆ alkynyl or Z-substituted alkynyl, C₃-C₈ cycloalkyl or Z-substituted cycloalkyl, C₆-C₁₀ aryl or Z-substituted aryl, 4-15-membered heterocyclic radical or Z-substituted heterocyclic radical, 5-15-membered heteroaryl or Z-substituted heteroaryl, or C₁-C₆ alkoxy or Z-substituted C₁-C₆ alkoxy, or R⁶ and R⁷ together with the atom to which they are bonded to form a 3-7-membered heterocyclic radical or Z-substituted 3-7-membered heterocyclic radical;
a is 0, 1, 2 or 3;
X is C or N;
Y is O or S;
Cx is selected from the group consisting of C₆-C₁₀ aryl or Z-substituted aryl, 4-15-membered heterocyclic radical or Z-substituted 4-15-membered heterocyclic radical, 5-15-membered heteroaryl or Z-substituted heteroaryl, 7-15-membered fused ring or Z-substituted fused ring, and -CONR⁶R⁷, -SO₂NR⁶R⁷, -SO₂R⁶, - OCOO-R⁶, -COOR⁶, -NR⁶COR⁷, -OCOR⁶, -NR⁶SO₂R⁷, -NR⁶SO₂NR⁶R⁷, -COR⁶, -NR⁶CONR⁶R⁷ substituted C₆-C₁₀ aryl, 4-15-membered heterocyclic radical, 5-15-membered heteroaryl, and 7-15-membered fused ring; and R⁶ and R⁷ together with N to form or not to form a 4-8-membered Z-substituted heterocycle;
L is selected from the group consisting of -O-, -S-, -OCOO-, -NR⁶CO-, -OCO-, -NR⁶SO₂-, -OCONR⁶-, quaternary ammonium, and sulfonate group -OSO₂-;
Cy is selected from the group consisting of hydrogen, deuterium, C₆-C₁₀ aryl or Z-substituted aryl, 4-15-membered heterocyclic or Z-substituted heterocyclic, 5-15-membered heteroaryl or Z-substituted heteroaryl, 7-15-membered fused ring or Z-substituted fused ring, C₁-C₆ alkyl or Z-substituted alkyl, C₂-C₆ alkenyl or Z-substituted alkenyl, C₂-C₆ alkynyl or Z-substituted alkynyl, and C₃-C₈ cycloalkyl or Z-substituted C₃-C₈ cycloalkyl;
or
Cy is selected from the group consisting of 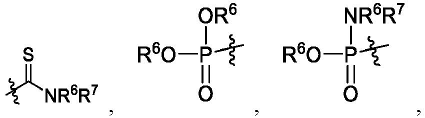 and 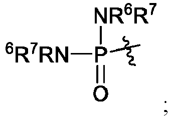
or Cy is selected from the group consisting of a 5-10-membered ring group formed by the two OR⁶ and the P atom in 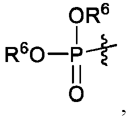 a 5-10-membered ring group formed by the OR⁶ and the NR⁶R⁷ together with the P atom in 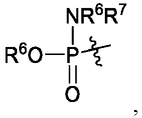 and a 5-10-membered ring group formed by the two NR⁶R⁷ and the P atom in 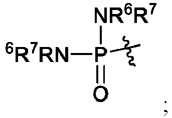
and -L-Cy excludes the residues of these phosphoramidate alkylating agents after losing a H atom: - P(Z¹)(NR⁹CH₂CH₂X¹)₂, -P(Z¹)(NR⁹₂)(N(CH₂CH₂X¹)2), -P(Z¹)(N(CH₂CH₂X¹))₂ or-P(Z¹)(N(CH₂CH₂X¹)₂)₂, each R⁹ is independently hydrogen or C₁-C₆ alkyl, or two R9 together with the nitrogen atom to which they are bonded to form a 5-7-membered heterocyclyl, Z¹ is O or S, X¹ is Cl, Br or OMs, and -L-Cy excludes -OH or -SH;
the substituent Z is a halogen atom, cyano or isocyanato, hydroxyl, mercapto, amino, oximido, hydrazono, OTs, OMs, C₁-C₃ alkyl or substituted alkyl, C₁-C₃ alkoxy or substituted alkoxy, C₂-C₃ alkenyl or substituted alkenyl, C₂-C₃ alkynyl or substituted alkynyl, C₃-C₈ cycloalkyl or substituted cycloalkyl, an aromatic ring, heterocyclic ring, a heteroaromatic ring and fused ring or a substituted aromatic ring, heterocyclic ring, heteroaromatic ring and fused ring, and the pattern of substitution being mono-substitution or geminal disubstitution;
Cz group is a C-, P-, S-containing group which can be hydrolyzed by hydrolytic enzymes such that corresponding C-N, P-N or S-N bond is cleaved.

2. The compound according to claim 1, wherein the compound is selected from the compounds of formula I, 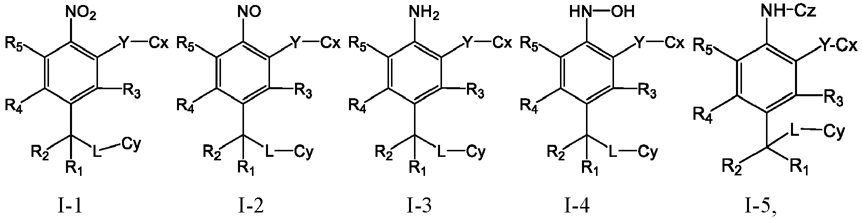 wherein,
I-5 is a prodrug that can be converted *in vivo* to the above compound I-3,
R₁ and R₂ are each independently hydrogen, deuterium, aryl or Z-substituted aryl, heteroaryl or Z-substituted heteroaryl, C₁-C₆ alkyl or Z-substituted alkyl, C₂-C₆ alkenyl or Z-substituted alkenyl, C₂-C₆ alkynyl or Z-substituted alkynyl, or C₃-C₈ cycloalkyl or Z-substituted cycloalkyl;
R₃ is hydrogen, halogen, cyano or isocyanato, hydroxyl, mercapto, amino, oximido, hydrazono, OTs, OMs, C₁-C₆ alkyl or Z-substituted alkyl, C₂-C₆ alkenyl or Z-substituted alkenyl, C₂-C₆ alkynyl or Z-substituted alkynyl, C₃-C₈ cycloalkyl or Z-substituted cycloalkyl, C₆-C₁₀ aryl or Z-substituted aryl, 4-15-membered heterocyclic radical or Z-substituted heterocyclic radical, 5-15-membered heteroaryl or Z-substituted heteroaryl, or C₁-C₆ alkoxy or Z-substituted C₁-C₆ alkoxy; or R₃ is -CONR⁶R⁷, -SO₂NR⁶R⁷, -SO₂R⁶, -OCO-R⁶, -OCOO-R⁶, -COOR⁶, -NR⁶COR⁷, -NR⁶SO₂R⁷, or -NR⁶CONR⁶R⁷, and R⁶ and R⁷ together with the N to form or not to form a 4-8-membered Z-substituted heterocyclic ring;
R₄ and R₅ are each independently hydrogen, halogen, cyano or isocyano, hydroxy, mercapto, amino, oximido, hydrazono, OTs, OMs, C₁-C₆ alkyl or Z-substituted alkyl, C₂-C₆ alkenyl or Z-substituted alkenyl, C₂-C₆ alkynyl or Z-substituted alkynyl, C₃-C₈ cycloalkyl or Z-substituted cycloalkyl, C₆-C₁₀ aryl or Z-substituted aryl, 4-15-membered heterocyclic radical or Z-substituted heterocyclic radical, 5-15-membered heteroaryl or Z-substituted heteroaryl, or C₁-C₆ alkoxy or Z-substituted C₁-C₆ alkoxy; or R₄ and R₅ are each -CONR⁶R⁷, - SO₂NR⁶R⁷, -SO₂R⁶, -OCOO-R⁶, -COOR⁶, -NR⁶COR⁷, -OCOR⁶, -NR⁶SO₂R⁷, or -NR⁶CONR⁶R⁷, or R₄ and R₅ together with the atom on a benzene ring to which they are bonded to form a 7-15-membered fused ring or a Z-substituted fused ring, and R₆ and R₇ together with N to form or not to form a 4-8-membered Z-substituted heterocyclic ring;
R₆ and R₇ are each independently hydrogen, cyano or isocyanato, C₁-C₆ alkyl or Z-substituted alkyl, C₂-C₆ alkenyl or Z-substituted alkenyl, C₂-C₆ alkynyl or Z-substituted alkynyl, C₃-C₈ cycloalkyl or Z-substituted cycloalkyl, C₆-C₁₀ aryl or Z-substituted aryl, 4-15-membered heterocyclic radical or Z-substituted heterocyclic radical, 5-15-membered heteroaryl or Z-substituted heteroaryl, or C₁-C₆ alkoxy or Z-substituted C₁-C₆ alkoxy; or R⁶ and R⁷ groups together with the atom to which they are bonded to form a 3-7-membered heterocyclyl or a Z-substituted 3-7-membered heterocyclyl, and R⁶ and R⁷ together with N to form or not to form a 4-8-membered Z-substituted heterocyclic ring;
Y is O or S;
Cx is selected from the group consisting of C₆-C₁₀ aryl or Z-substituted aryl, 4-15-membered heterocyclic radical or Z-substituted 4-15-membered heterocyclic radical, 5-15-membered heteroaryl or Z-substituted heteroaryl, 7-15-membered fused ring or Z-substituted fused ring, and -CONR⁶R⁷, -SO₂NR⁶R⁷, -SO₂R⁶, - OCOO-R⁶, -COOR⁶, -NR⁶COR⁷, -OCOR⁶, -NR⁶SO₂R⁷, -NR⁶SO₂NR⁶R⁷, -COR⁶, -NR⁶CONR⁶R⁷ substituted C₆-C₁₀ aryl, 4-15-membered heterocyclic radical, 5-15-membered heteroaryl, 7-15-membered fused ring, and R⁶ and R⁷ together with N to form or not to form a 4-8-membered Z-substituted heterocycle;
L is selected from the group consisting of -O-, -S-, -OCOO-, -NR⁶CO-, -OCO-, -NR⁶SO₂-, -OCONR⁶-, quaternary ammonium, and sulfonate group -OSO₂-;
Cy is selected from the group consisting of hydrogen, deuterium, C₆-C₁₀ aryl or Z-substituted aryl, 4-15-membered heterocyclic radical or Z-substituted heterocyclic radical, 5-15-membered heteroaryl or Z-substituted heteroaryl, 7-15-membered fused ring or Z-substituted fused ring, C₁-C₆ alkyl or Z-substituted alkyl, C₂-C₆ alkenyl or Z-substituted alkenyl, C₂-C₆ alkynyl or Z-substituted alkynyl, or C₃-C₈ cycloalkyl or Z-substituted C₃-C₈ cycloalkyl;
or
Cy is selected from the group consisting of 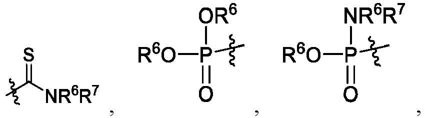 and 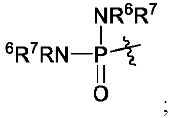
or Cy is selected from the group consisting of a 5-10-membered ring group formed by the two OR⁶ and the P atom in 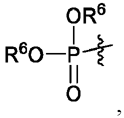 a 5-10-membered ring group formed by the OR⁶ and the NR⁶R⁷ together with the P atom in 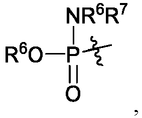 and a 5-10-membered ring group formed by the two NR⁶R⁷ and the P atom in 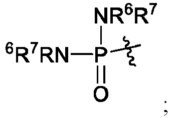
the substituent Z is a halogen atom, cyano or isocyanato, hydroxyl, mercapto, amino, oximido, hydrazono, OTs, OMs, C₁-C₃ alkyl or substituted alkyl, C₁-C₃ alkoxy or substituted alkoxy, C₂-C₃ alkenyl or substituted alkenyl, C₂-C₃ alkynyl or substituted alkynyl, C₃-C₈ cycloalkyl or substituted cycloalkyl, an aromatic ring, heterocyclic ring, a heteroaromatic ring and fused ring or a substituted aromatic ring, heterocyclic ring, heteroaromatic ring and fused ring, and the pattern of substitution being mono-substitution or geminal disubstitution;
Cz group is a C-, P-, S-containing group which can be hydrolyzed by hydrolytic enzymes such that corresponding C-N, P-N or S-N bond is cleaved.

3. The compound according to claims 1-2, wherein,
R₁ and R₂ are each independently hydrogen, deuterium, C₁-C₆ alkyl or Z-substituted alkyl, C₂-C₆ alkenyl or Z-substituted alkenyl, C₂-C₆ alkynyl or Z-substituted alkynyl, or C₃-C₈ cycloalkyl or Z-substituted cycloalkyl.

4. The compound according to claim 3, wherein,
R₁ and R₂ are each independently hydrogen, deuterium, or methyl.

5. The compound according to claims 1-4, wherein,
R₃, R₄ and R₅ are each independently hydrogen.

6. The compound according to claims 1-5, wherein,
Cx is -CONR⁶R⁷ substituted phenyl, and R⁶ and R⁷ together with N to form or not to form a 4-8-membered Z-substituted heterocyclic ring.

7. The compound according to claims 1-6, wherein,
L is selected from -O- or -S-.

8. The compound according to claims 1-7, wherein,
Cy is selected from the group consisting of C₆-C₁₀ aryl or halo-substituted aryl, 4-15-membered heterocyclic or halo-substituted heterocyclic, 5-15-membered heteroaryl or halo-substituted heteroaryl, and 7-15-membered fused ring or halo-substituted fused ring.

9. The compound according to claim 8, wherein,
Cy is selected from the group consisting of fluorophenyl, difluorophenyl, and trifluorophenyl.

10. The compound according to claims 1-7, wherein,
Cy is selected from the group consisting of 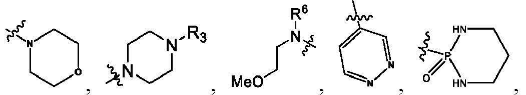 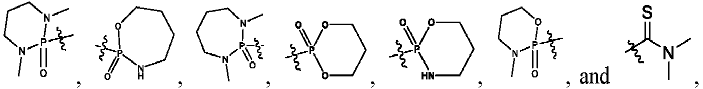 or Z-substituted 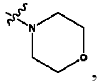 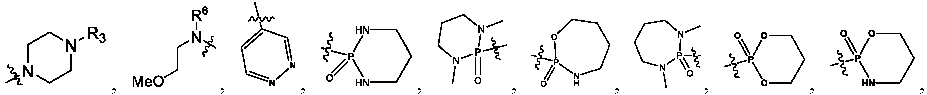 and

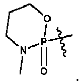

11. The compound according to claims 1-2, wherein,
Cz is selected from -COR⁶, or -COOR⁶.

12. The compound according to claims 1-10, wherein,
-NH-Cz is a phosphamido group.

13. The compound according to claims 1-10, wherein the compound is selected from compounds of the following structural formulae: 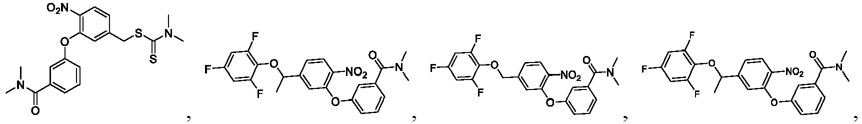 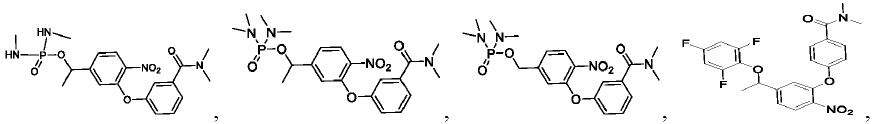 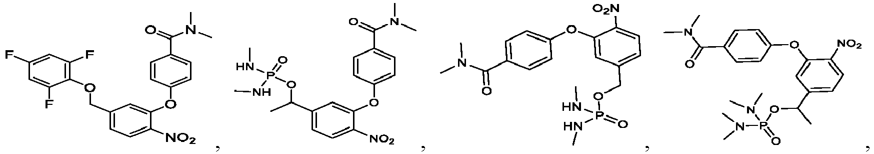 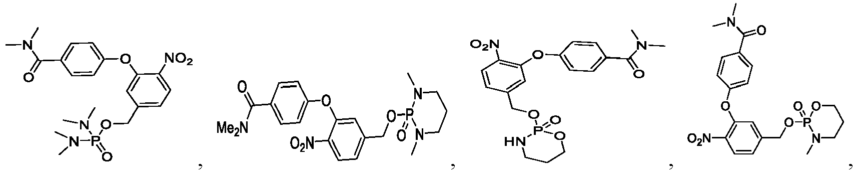 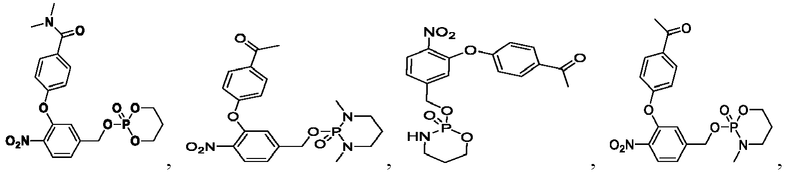 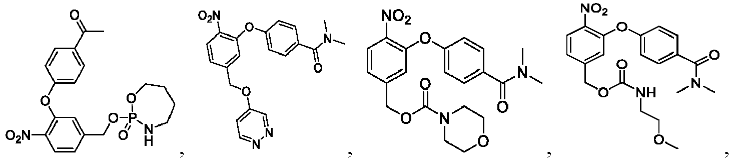 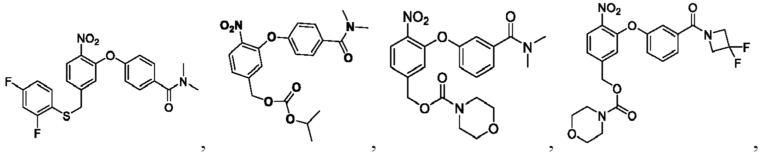 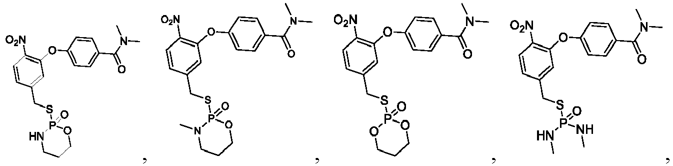 or

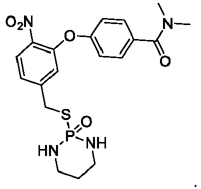

14. The compound according to any one of claims 1 to 13, wherein the salt is a basic salt or an acid salt and the solvate is a hydrate or an alcoholate.

15. A medicament containing the compound according to any one of claims 1 to 13, and the pharmaceutically acceptable salt, or the solvate, or the isotopically substituted compound thereof.

16. The medicament according to claim 15, wherein the medicament is used for preventing or treating diseases/conditions comprising endometriosis, uterine leiomyoma, uterine hemorrhagic disorders, dysmenorrhea, prostatic hyperplasia, acne, seborrhea, alopecia, premature sexual maturation, polycystic ovary syndrome, chronic obstructive pulmonary disease COPD, obesity, inflammatory pain, cancer, inflammation, or cancer pain.

17. The medicament according to claim 16, wherein the cancer comprises prostate cancer, primary prostate cancer, advanced prostate cancer, metastatic prostate cancer, hormone naive prostate cancer, refractory prostate cancer, castration-resistant prostate cancer CRPC, breast cancer, lung cancer, endometrial cancer, renal cell carcinoma, bladder cancer, non-Hodgkin lymphoma, acute myeloid leukemia AML, T-cell acute lymphoblastic leukemia T-ALL, and leukemia.

18. Use of the compound according to any one of claims 1 to 13, and a pharmaceutically acceptable salt, or a solvate, or an isotopically substituted compound thereof, in the manufacture of a medicament for treating or preventing relevant disease/disorders, wherein the disease/disorders comprise endometriosis, uterine leiomyoma, uterine hemorrhagic disorders, dysmenorrhea, prostatic hyperplasia, acne, seborrhea, alopecia, premature sexual maturation, polycystic ovary syndrome, chronic obstructive pulmonary disease COPD, obesity, inflammatory pain, cancer, inflammation, or cancer pain.

19. A medicament for enhancing sensitivity to radiation therapy for cancers or tumors, wherein the medicament comprises the compound according to any one of claims 1 to 13, and the pharmaceutically acceptable salt, or the solvate, or the isotopically substituted compound thereof, wherein the medicament is used for increasing the efficacy of radiation therapy when a patient with a cancer or a tumor is resistant to radiation therapy, or treating a patient with a cancer or a tumor who is resistant to radiation therapy.

20. A medicament for enhancing sensitivity to immunotherapy for cancers or tumors, wherein the medicament comprises the compound according to any one of claims 1 to 13, and the pharmaceutically acceptable salt, or the solvate, or the isotopically substituted compound thereof, wherein the medicament is used for increasing the efficacy of radiation therapy when a patient with a cancer or a tumor is resistant to immunotherapy, or treating a patient with a cancer or a tumor who is resistant to immunotherapy.

21. A medicament for enhancing sensitivity to chemotherapy for cancers or tumors, wherein the medicament comprises the compound according to any one of claims 1 to 13, and the pharmaceutically acceptable salt, or the solvate, or the isotopically substituted compound thereof, wherein the medicament is used for increasing the efficacy of chemotherapy when a patient with a cancer or a tumor is resistant to chemotherapy, or treating a patient with a cancer or a tumor who is resistant to chemotherapy.

22. A medicament for enhancing sensitivity to chemotherapy for cancers or tumors, wherein the medicament comprises the compound according to any one of claims 1 to 13, and the pharmaceutically acceptable salt, or the solvate, or the isotopically substituted compound thereof, wherein the medicament is used for increasing the efficacy of chemotherapy when a patient with a cancer or a tumor is resistant to chemotherapy, or treating a patient with a cancer or a tumor who is resistant to chemotherapy, and wherein the medicament used in the chemotherapy contains daunorubicin or cytarabine.

23. Use of the compound according to any one of claims 1 to 13, and the pharmaceutically acceptable salt, or the solvate, or the isotopically substituted compound thereof for enhancing the sensitivity to radiation therapy for a cancer or a tumor, or for enhancing the sensitivity to chemotherapy for a cancer or a tumor using a medicament containing daunorubicin or cytarabine.

24. Use of the compound according to any one of claims 1 to 13, and the pharmaceutically acceptable salt, or the solvate, or the isotopically substituted compound thereof as an AKR1C3 enzyme inhibitor.

25. Use of the compound according to any one of claims 1 to 13, and the pharmaceutically acceptable salt, or the solvate, or the isotopically substituted compound thereof in the manufacture of a medicament, wherein the medicament is an AKR1C3 enzyme inhibitor.

26. Use of the compound according to any one of claims 1 to 13, and the pharmaceutically acceptable salt, or the solvate, or the isotopically substituted compound thereof in the manufacture of a medicament, wherein the medicament is used for enhancing the efficacy of radiation therapy when a patient with a cancer or a tumor is resistant to radiation therapy, or for treating a patient with a cancer or a tumor who is resistant to radiation therapy.

27. Use of the compound according to any one of claims 1 to 13, and the pharmaceutically acceptable salt, or the solvate, or the isotopically substituted compound thereof in the manufacture of a medicament, wherein the medicament is used for enhancing the efficacy of radiation therapy when a patient with a cancer or a tumor is resistant to immunotherapy, or for treating a patient with a cancer or a tumor who is resistant to immunotherapy.

28. Use of the compound according to any one of claims 1 to 13, and the pharmaceutically acceptable salt, or the solvate, or the isotopically substituted compound thereof in the manufacture of a medicament, wherein the medicament is used for enhancing the efficacy of chemotherapy when a patient with a cancer or a tumor is resistant to chemotherapy, or for treating a patient with a cancer or a tumor who is resistant to chemotherapy.

29. Use of the compound according to any one of claims 1 to 13, and the pharmaceutically acceptable salt, or the solvate, or the isotopically substituted compound thereof in the manufacture of a medicament, wherein the medicament is used for enhancing the efficacy of chemotherapy when a patient with a cancer or a tumor is resistant to chemotherapy, or for treating a patient with a cancer or a tumor who is resistant to chemotherapy, wherein the medicament used in the chemotherapy contains daunorubicin or cytarabine.
